(19) Europäisches Patentamt
European Patent Office
Office européen des brevets

(11) Publication number: 0 332 392 B1

(12) # EUROPEAN PATENT SPECIFICATION

(45) Date of publication of patent specification: 07.12.94 (51) Int. Cl.5: C07D 319/06, C09K 19/34

(21) Application number: 89302252.5

(22) Date of filing: 07.03.89

(54) **Dioxane liquid crystal compounds.**

(30) Priority: 09.03.88 JP 53805/88

(43) Date of publication of application:
13.09.89 Bulletin 89/37

(45) Publication of the grant of the patent:
07.12.94 Bulletin 94/49

(84) Designated Contracting States:
DE FR GB

(56) References cited:
DD-A- 227 706
DD-A- 227 719

IBM TECHNICAL DISCLOSURE BULLETIN, vol. 30, no. 8, January 1988, page 34,Armonk, NY, US; "Liquid crystals"

(73) Proprietor: Showa Shell Sekiyu Kabushiki Kaisha
7-3, Marunouchi 2-chome
Chiyoda-ku
Tokyo 100 (JP)

(72) Inventor: Suzuki, Yoshiichi c/o Showa Shell Sekiyu K.K.
7-3, Marunouchi 2-chome,
Chiyoda-ku
Tokyo (JP)
Inventor: Hagiwara, Takashi c/o Showa Shell Sekiyu K.K.
7-3, Marunouchi 2-chome,
Chiyoda-ku
Tokyo (JP)
Inventor: Numazawa, Koichi c/o Showa Shell Sekiyu K.K.
7-3, Marunouchi 2-chome
Chiyoda-ku
Tokyo (JP)

(74) Representative: Ouest, Barry et al
Wilson, Gunn, M'Caw & Co.,
41-51 Royal Exchange,
Cross Street
Manchester M2 7BD (GB)

EP 0 332 392 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION AND RELATED ARTS

The invention relates to liquid crystal compounds having a dioxane ring, and more particularly smectic and ferroelectric dioxane liquid crystal compounds showing a tristable phase.

Liquid crystal display elements have been widely used in twisted nematic (TN) mode, guest-host (GH) mode and so on owing to the excellent properties thereof such as low voltage actuability, low energy consumption, displayability in a thin structure and so on.

The liquid crystal display elements of the nematic type, however, are disadvantageous in that slow speed of response in the order of several m sec-several decades of m sec so that there are considerable limitations in the applied uses thereof.

In order to solve the problem referred to above, a super twisted nematic (STN) mode has been developed, but it is still unsatisfactory in that it is necessary to precisely control a cell gap and a tilt angle and in that the time response is still low, despite of that display properties such as a display contrast and a visual field angle have been considerably improved.

In order to comply with demands for novel liquid crystal displays superior in the time of response, ferroelectric liquid crystals were developed so as to provide liquid crystal devices having the far higher optical response time in the order of μsec.

In the year of 1975, p-decyloxybenzilidene-p-amino-2-methylbutylcinamate (DOBAMBC) was synthesized as the ferroelectric liquid crystal by Meyer et al., and in the year of 1980 the high speed switching and memory properties of DOBAMBC were confirmed by Clark and Longwall (N.A. Clark et al., Appl. Phys. Lett. 36, 899 (1980)) so as to attract attentions as an epoch-making liquid crystal element material capable of realizing the moving image display of a large picture in the simple matrix mode.

However, there were still technical problems so as to actually use the above ferroelectric liquid crystal. Above all, satisfactory ferroelectricity at the room temperature and satisfactory control or orientation of the liquid crystal molecules which is necessary for the display device could not be attained.

The inventors et al have found some ferroelectric liquid crystals showing tristable phase $S_{(s)}^*$ which has definite threshhold and hysterisis properties relative to driving voltage so as to realize large moving picture display according to the simple matrix mode and a novel electroptical shutter owing to the particular electroclinic effect thereof, which is disclosed in A.D.L. Chandani, T. Hagiwara, Y. Suzuki, et al., Japan J. of Appl. Phys., 27 (5), 729-732 (1988), and JP-A Sho 63 (1988)-21159 and 21160.

### SUMMARY OF THE INVENTION

It is an object of the invention, thus, to provide further novel ferroelectric liquid crystal compounds showing tristable phase $S_{(s)}^*$ in a wider range of temperatures inclusive of the room temperature.

The other objects and advantageous effects of the invention will be appreciated by those skilled in the art by studying the detailed explanation to be given hereafter.

The above objects can be attained by a certain scope of compounds, each having a dioxane ring as the heterocyclic ring as definitely defined in the Claims.

### DESCRIPTION OF THE DRAWINGS

Fig. 1 shows an infrared absorption spectrum (KBr) of (R)-(+)-4'-(1-trifluoromethylnonyloxycarbonyl)-1-phenyl 4-(5-n-pentyl-trans-1,3-dioxane-2-yl)benzoate, the objective compound in Example 1,

Fig. 2 shows the spectrum of 4'-(1-trifluoromethylheptyloxycarbonyl)phenyl 4-(5-n-nonyl-trans-1,3-dioxane-2-yl)benzoate, the objective compound in Example 2,

Fig. 3 shows the spectrum of (R)-(+)-4-(1-trifluoromethyl-2-ethoxycarbonyloxycarbonyl) phenyl 4-(5-n-pentyl-trans-1,3-dioxane-2-yl)benzoate, the objective compound in Example 3,

Fig. 4 shows an optical response wave form of the objective compound in Example 3 according to the electroclinic effect in the $S_A$ phase, and

Fig. 5 shows an optical response wave form of the objective compound in Example 2 in the $S_{(s)}^*$ phase.

DESCRIPTION OF PREFERRED EMBODIMENTS OF THE INVENTION

The invention relates to liquid crystal compounds represented by the general formula (I)

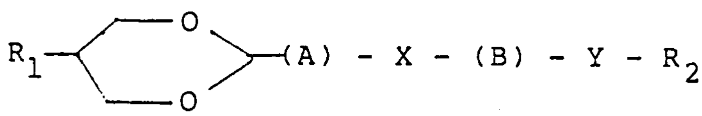

(I)

$R_1$ means an alkyl, alkoxy, alkyloxycarbonyl or alkylcarbonyloxy group having 1 - 20 carbon atoms, preferably $C_nH_{2n+1}$, $C_nH_{2n+1}O$,

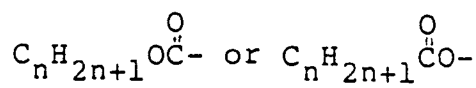

or

wherein n is an integer of 5 - 10, and more preferably pentyl($C_5H_{15}$), octyl($C_8H_{17}$), nonyl($C_9H_{19}$) or decyl-($C_{10}H_{21}$).

(A) means

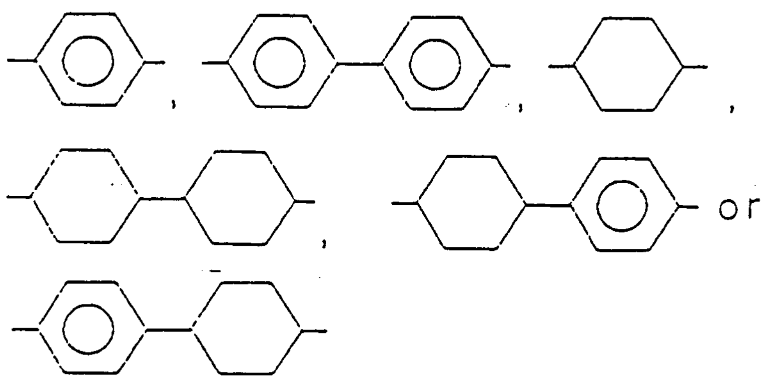

or

X means

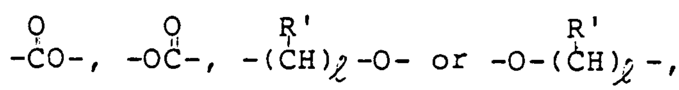

or

wherein R' is hydrogen or a lower alkyl such as methyl and methyl, and ℓ is an integer of 1 - 16, preferably

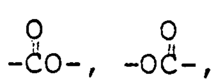

$-OCH_2-$, $-OCH_2CH_2-$, $OCH_2CH_2CH_2-$, $-CH_2CH_2O-$or $-CH_2CH_2CH_2O-$, and more preferably

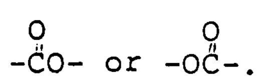

or

(B) means

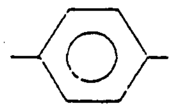, 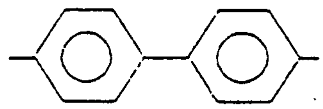, 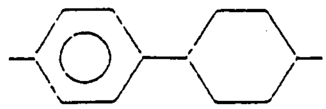 or

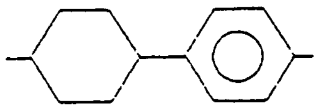

Y means

$$-\overset{O}{\overset{\|}{C}}O-,$$

-O-, -S- or $-CH_2O-$, and preferably

$$-\overset{O}{\overset{\|}{C}}O.$$

$R_2$ means

$$-(CH_2)m-\overset{Z}{\underset{*}{C}}H-R_3 \text{ or } -(CH_2)m-\overset{Z}{\underset{*}{C}}H-CH_2-\overset{O}{\overset{\|}{C}}O-R_3$$

in which Z means $CF_3$, $C_2F_5$, $CHF_2$, $CH_2F$, $CClF_2$, $CCL_2F$, $CF_3CCl_2$ or $C_3F_7$, and preferably $CF_3$, $C_2F_2$ or $C_3F_7$; $R_3$ means a straight or branched alkyl, aralkyl, alkoxyalkyl, alkoxycarbonyl or alkoxycarbonylalkyl group of 1 - 20 carbon atoms, or a substituted vinyl, substituted cyclopropyl or substituted epoxy group; and m is an integer of 0 - 20 and preferably 0 - 10.

$R_2$ preferably means

$$-(CH_2)m-\overset{Z}{\underset{*}{C}}H-C_nH_{2n+1} \text{ or}$$

$$-(CH_2)m-\overset{Z}{\underset{*}{C}}H-CH_2-\overset{O}{\overset{\|}{C}}O-C_nH_{2n+1},$$

in which m and n mean respectively an integer of 0 - 10. The preferable group, $C_nH_{2n+1}$ is pentyl, hexyl, octyl, nonyl or decyl.

More definitely, $R_2$ is a radical derived from any of the following optically active alcohols;
1,1,1-trifluoro-2-$C_6$ - $C_{16}$ alkanol,
1,1-difluoro-2-$C_6$ - $C_{16}$ alkanol,
1-monofluoro-2-$C_6$ - $C_{16}$ alkanol,
1,1,1,2,2-pentafluoro-3-$C_6$ - $C_{16}$ alkanol,
1-monofluoro-1,1-dichloro-2-$C_6$ - $C_{16}$ alkanol,
1,1,1-trichloro-2-$C_6$ - $C_{16}$ alkanol,
1,1-difluoro-1-monochloro-2-$C_6$ - $C_{16}$ alkanol,
1,1,1-trifluoromethyl-1-phenylmethanol,
1,1,1-trifluoromethyl-2-phenylethanol,

1,1,1-trifluoromethyl-3-phenylpropanol,
1,1,1-trifluoro-3-decene-2-al,
1,1,1-trifluoro-3-heptene-2-al,
methyl-4,4,4-trifluoro-3-hydroxybutylate,
ethyl-4,4,4-trifluoro-3-hydroxybutylate,
propyl-4,4,4-trifluoro-3-hydroxybutylate,
butyl-4,4,4-trifluoro-3-hydroxybutylate,
pentyl-4,4,4-trifluoro-3-hydroxybutylate,
hexyl-4,4,4-trifluoro-3-hydroxybutylate,
heptyl-4,4,4-trifluoro-3-hydroxybutylate,
octyl-4,4,4-trifluoro-3-hydroxybutylate,
nonyl-4,4,4-trifluoro-3-hydroxybutylate, and
decyl-4,4,4-trifluoro-3-hydroxybutylate.

The haloalcohols may be manufactured according to disclosures in e.g. "J. Org. Chem." 52, (15), 3211 (1987) and "Sience" 56, (9), 531 (1986).

Particularly preferable liquid crystal compounds of the invention are represented by the formula (I), in which $R_1$ means an alkyl, alkoxy, alkylalkoxycarbonyl or alkylcarbonyloxy group of 4 - 20 carbon atoms, preferably $C_nH_{2n+1}$, $C_nH_{2n+1}O$,

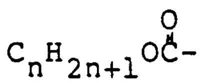 or 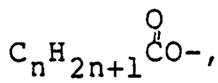,

and more preferably a straight chain alkyl or $C_nH_{2n+1}$ in which n is an integer of 5 - 10;

(A) means

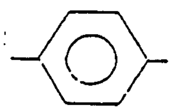, 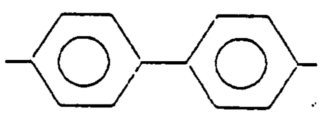, 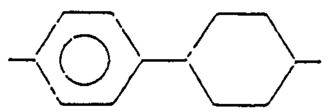 or

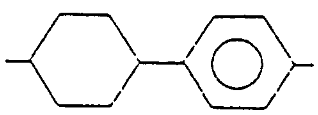

X means

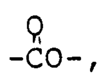, 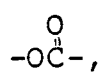,

$-CH_2O-$, -O- or $-OCH_2-$, preferably

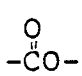 or 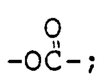;

(B) means

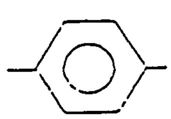 or 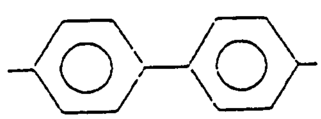

Y means

$$-\overset{O}{\overset{\|}{C}}O-,$$

-O-, -S- or $-CH_2O-$; and

$R_2$ means

$$-(CH_2)m-\overset{Z}{\underset{*}{C}}-R_3 \quad or \quad -(CH_2)m-\overset{Z}{\underset{*}{C}}H-CH_2-\overset{O}{\overset{\|}{C}}O-R_3$$

wherein Z is $CF_3$, $C_2F_5$, $CHF_2$, $CH_2F$, $CClF_2$, $CCl_2F$, $CF_3CCl_2$ or $C_3F_7$, preferably $CF_3$, $C_2F_5$ or $C_3F_7$; $R_3$ means a straight or branched alkyl or 3 - 15 carbon atoms; and m is an integer of 0 - 15, preferably 0 - 10, and $R_2$ more preferably means

$$-(CH_2)m-\overset{Z}{\underset{*}{C}}H-C_nH_{2n+1} \quad or$$

$$-(CH_2)m-\overset{Z}{\underset{*}{C}}H-CH_2-\overset{O}{\overset{\|}{C}}O-R_3$$

wherein $C_nH_{2n+1}$ is pentyl, hexyl, octyl, nonyl or decyl and derived from the optically active alcohols comprising 1,1,1-trifluoro-2-$C_6$ - $C_{16}$ alkanol; 1,1-difluoro-2-$C_6$ - $C_{16}$ alkanol; 1-monofluoro-2-$C_6$ - $C_{16}$ alkanol; 1,1,1,2,2-pentafluoro-3-$C_6$ - $C_{16}$ alkanol; 1-monofluoro- 1,1-dichloro-2-$C_6$ - $C_{16}$ alkanol; 1,1,1-trichloro-2-$C_6$ - $C_{16}$ alkanol; and 1,1-difluoro-1-1-monochloro-2-$C_6$ - $C_{16}$ alkanol.

Among the novel liquid crystal compounds of the invention represented by the formula (I), when $R_1$ is an alkyl, alkoxy, alkyloxycarbonyl or alkylcarbonyloxy group of 5 - 20 carbon atoms, and preferably $C_nH_{2n+1}$ or $C_nH_{2n+1}O$, and more preferably $C_nH_{2n+1}$ in which n is and integer of 6 - 10

(A) is

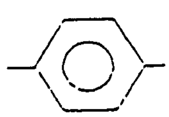
or
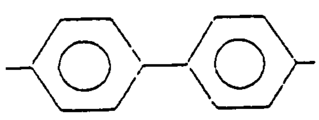

X is

$$-\overset{O}{\overset{\|}{C}}O- \quad or \quad -O\overset{O}{\overset{\|}{C}}-;$$

(B) is

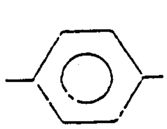
or
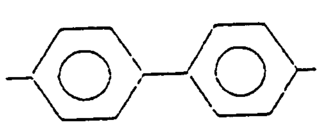

Y is

$$-\overset{O}{\overset{\|}{C}}O-;$$

$R_2$ is

$$-(CH_2)m-\overset{Z}{\underset{*}{C}}H-R_3$$

wherein Z means $CF_3$, $C_2F_5$, $CHF_2$, $CH_2F$, $CClF_2$, $CCl_2F$ or $CF_3CCl_2$ more preferably $CF_3$ or $C_2F_5$; $R_3$ means a straight or branched alkyl or 3 - 15 carbon atoms; and m is an integer of 0 - 3, preferably 0 or 1; $R_2$ is preferably

$$-(CH_2)m-\overset{Z}{\underset{*}{C}}H-C_nH_{2n+1}$$

in which $C_nH_{2n+1}$ preferably means pentyl, hexyl, octyl, nonyl or decyl, which is derived from 1,1,1-trifluoro-2-$C_6$ - $C_{16}$ alkanol, 1,1-difluoro-2-$C_6$ - $C_{16}$ alkanol, 1-monofluoro-2-$C_6$ - $C_{16}$ alkanol, 1,1,1,2,2-pentafluoro-3-$C_6$ - $C_{16}$ alkanol, 1-monofluoro-1,1-dichloro-2-$C_6$ - $C_{16}$ alkanol, 1,1,1-trichloro-2-$C_6$ - $C_{16}$ alkanol and 1,1-difluoro-1-1monochloro-2-$C_6$ -$C_{16}$ alkanol, those show the tristable liquid crystal phase.

The compounds of the invention may be manufactured as follows;

(A)

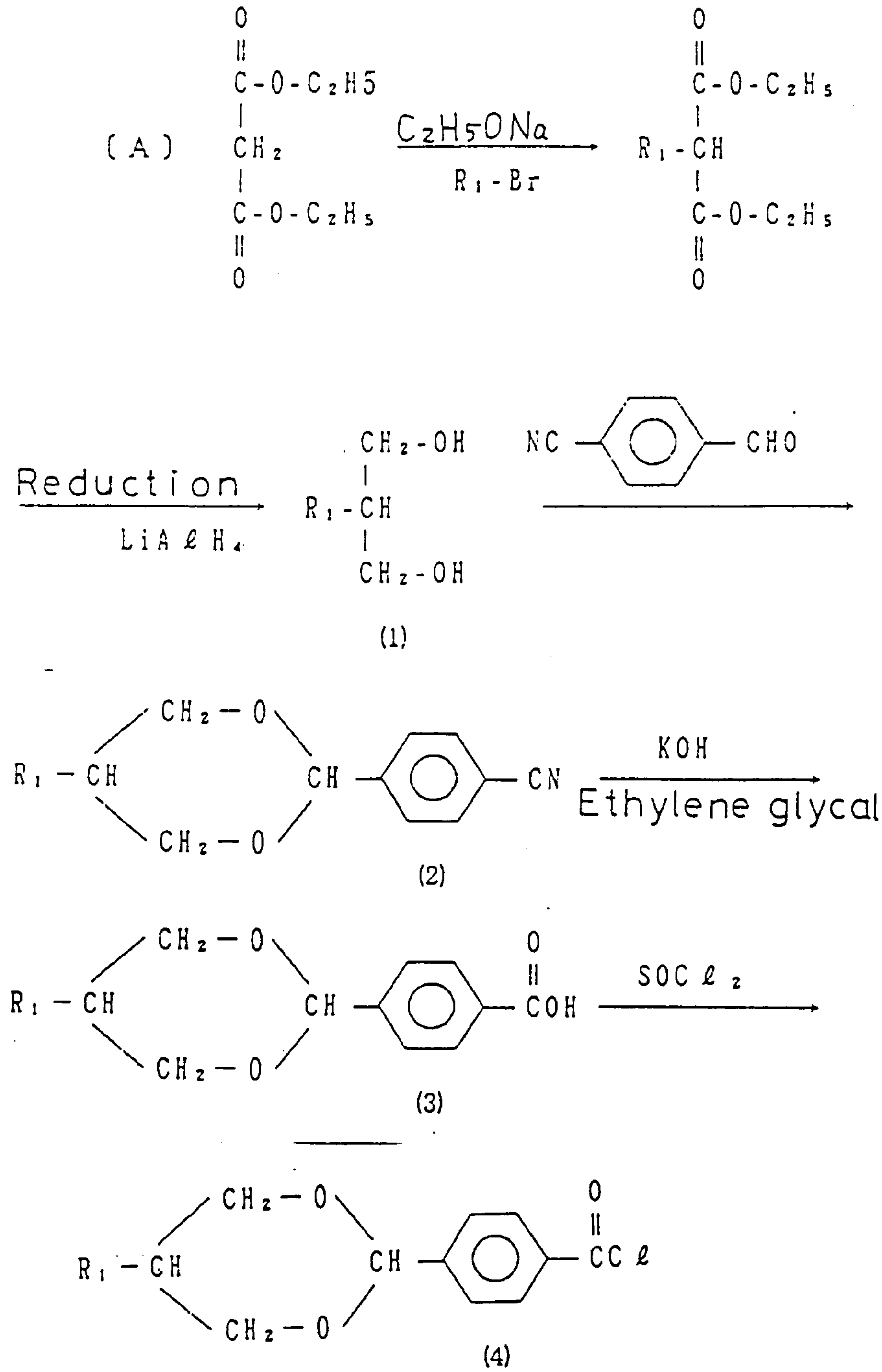


(1)

(2)

(3)

(4)

(B)

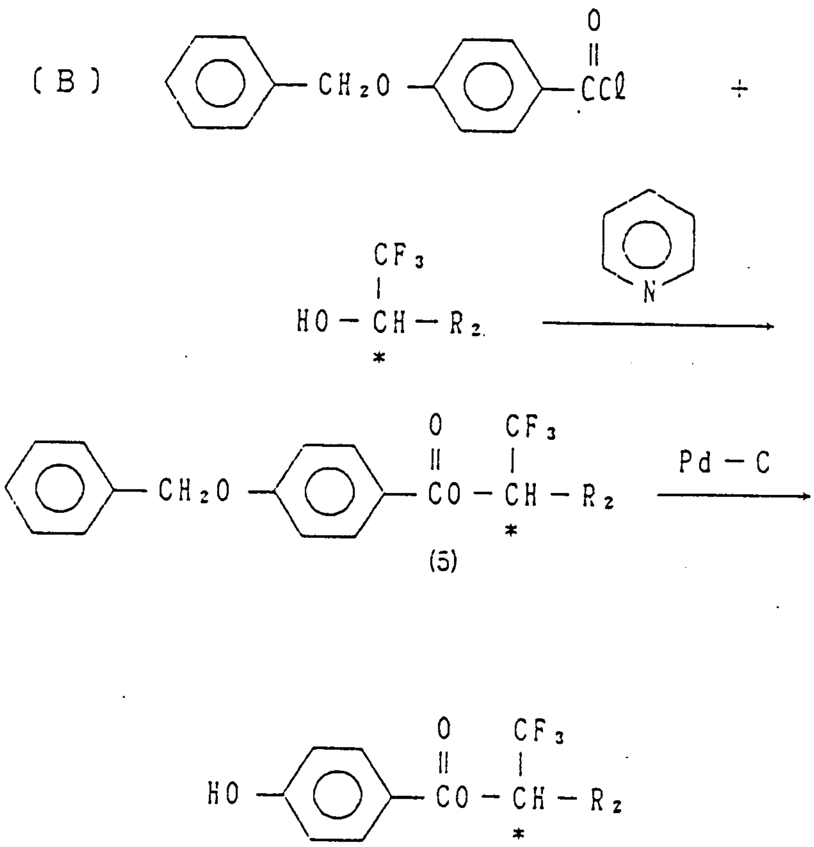


(5)

(6)

(C)

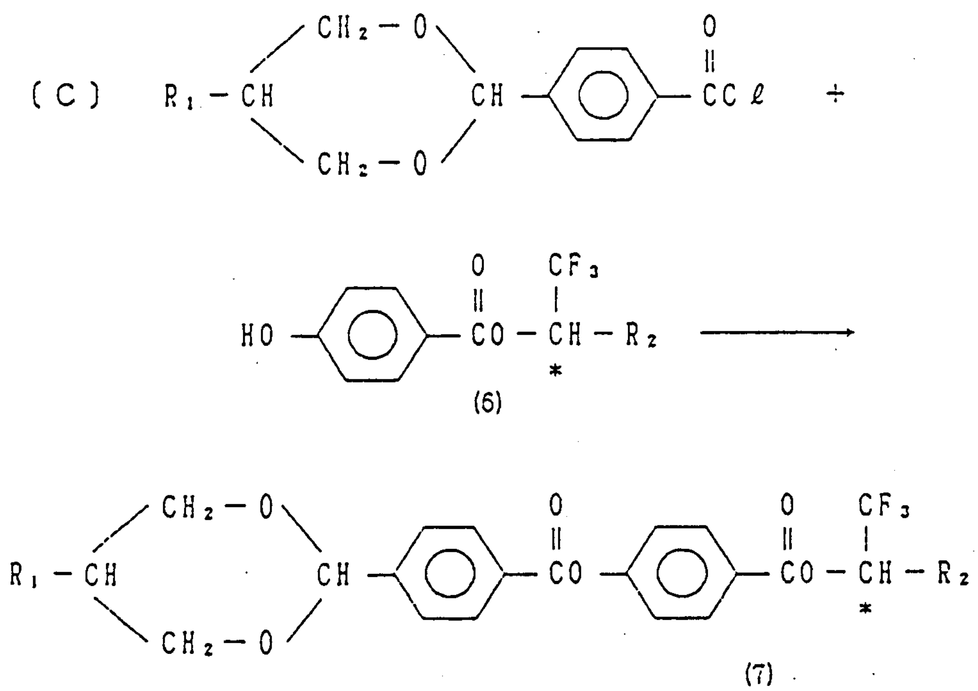

(6)

(7)

In the formulae $R_1$ and $R_2$ mean the same as above.

(a) Ethyl malonate is reacted with a sodium alcolate and an alkyl bromide to obtain a 2-alkyl-ethyl malonate, which is reduced in ether or tetrahydrofuran with hydrogenated lithium aluminum to obtain 2-alkyl-1,3-propanediol (1). The compound (1) is then reacted with 4-cyanobenzaldehyde to obtain 2-(4′-cyanophenyl)-5-alkyl-1,3-dioxane (2), of which cyano is hydrolyzed in the presence of caustic potash and

ethylene glycol to obtain 2-(4′-carboxyphenyl)-5-alkyl-1,3-dioxane (3). The compound (3) is treated with thionylchloride to obtain the corresponding chloride (4).

(B) Meanwhile, 4-benzyloxy-benzoic acid chloride is added with optically active 1-trifluoromethyl-1-alkanol in the presence of pyridine to obtain 1-trifluoromethyl-1-alkyl 4-benzyloxybenzoate (5), which is hydrogenated with using Pd/carbon as catalyst to obtain 1-trifluoromethyl-1-alkyl 4-hydroxybenzoate (6).

(C) Then the chloride (4) of the dioxane (3) is reacted with 1-trifluoromethyl-1-alkyl 4-hydroxybenzoate (6) to obtain the objective compound, 4′-(1-trifluoromethylalkyloxycarbonyl)-phenyl 4-(5-alkyl-1,3-dioxane-2-yl)benzoate (7).

Now some Examples are to be given, but the invention is of course not limited thereto.

Example 1

1) Synthesis of (R)-(+)-1-trifluoromethylnonyl 4-benzyloxybenzoate

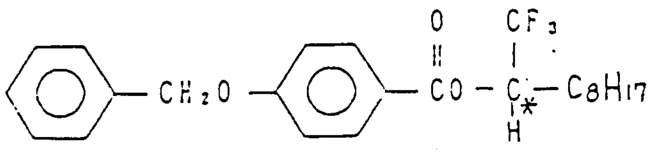

3.6 g of 4-Benzyloxy-benzoic acid-chloride and 2.2g of (R)-(+)-1,1,1-trifluoro-2-decanol ($[\alpha]_d = 22.3°$) were added in 35 ml of pyridine to be stirred at the room temperature for 12 hours and poured into ice-water and extracted with methylene chloride. The methylene chloride phase was washed successively with 1N sodium carbonate solution, water, diluted hydrochloric acid and water to recover the organic phase, which was dried over anhydrous magnesium sulphate, distilled to remove the solvent and subjected to toluene/silica gel chromatography to obtain the compound captioned above.

2) Synthesis of (R)-(+)-1-trifluoromethylnonyl 4-hydroxylbezoate

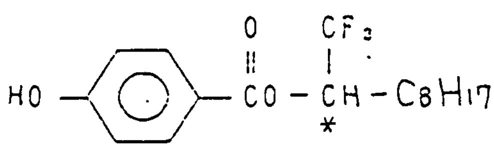

The compound obtained in 1) was dissolved in 50 ml of methanol, added with 0.2 g of 10% Pd/carbon catalyst and hydrogenated in an atmosphere of hydrogene under high pressure to obtain the captioned compound.

3) Synthesis of (R)-(+)-4′-(1-trifluoromethylnonyloxycarbonyl)-phenyl 4-(5-n-pentyl-trans-1,3-dioxane-2-yl)-benzoate

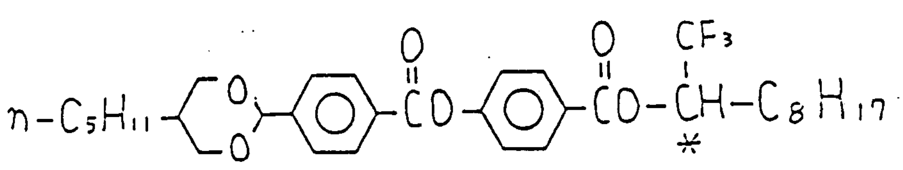

Thionyl in the excessive amount was reacted with 4-(5-n-pentyl-trans-1,3-dioxane-2-yl)-benzoic acid, which was obtained by alkaline hydrolysis of usually synthesized 4-(5-pentyl-1,3-dioxane-2-yl)benzonitrile in the amount of 2.2 g in ethylene glycol as solvent, under reflux for 6 hours and distilled for removing unreacted thionyl to obtain the acid chloride in the amount of 1.74 g.

(R)-(+)-1-trifluoromethylnonyl 4-hydroxybenzoate obtained in 2) in the amount of 1.52 g was reacted with the above acid chloride in the amount of 1.74 g in 20 ml of pyridine at the room temperature for a whole day and might. The reaction liquid was poured into ice-water and extracted with methylene chloride. The methylene chloride phase was successively washed with 1N sodium carbonate solution, water, diluted hydrochloric acid and water. The organic phase was dried over anhydrous magnesium sulphate and distilled for removing the solvent to obtain the crude objective compound.

The crude objective compound was subjected to toluene/silica gel chromatography to obtain the optically active compound ($[\alpha]_d = +28.9°$) in the amount of 1.1 g.

This liquid crystal compound of the invention shows following phase transition points;

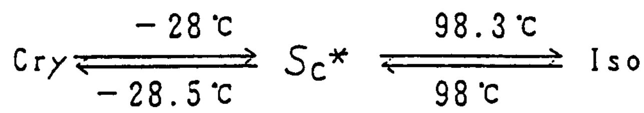


The infrared absorption spectrum (KBr) thereof is shown in Fig. 1 of the accompanying drawings.

Example 2

1) Synthesis of 1-trifluoromethylheptyl 4-benzyloxybenzoate

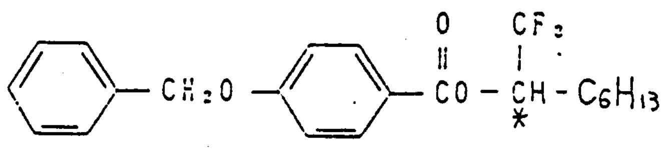

4-Benzyloxy-benzoic acid-chloride in the amount of 4.3 g was dissolved in 50 ml of methylene chloride, to which a solution of 2.9 g of optically active 1,1,1-trifluoro-2-octanol, 0.6 g of dimethylaminopyridine and 1.7 g of triethylamine in 50 ml of methylene chloride was gradually added under cooling with ice.

The mixture was held at the room temperature for reaction for a whole day and might. The reaction liquid was taken in ice-water and extracted with methylene chloride. The methylene chloride phase was successively washed with diluted hydrochloric acid, water, 1N sodium carbonate solution and water, dried over anhydrous magnesium sulphate, and distilled for removing the solvent to obtain the crude compound captioned above, which was treated according to toluene/silica gel chromatgraphy and recrystalized with ethanol to obtain the purified compound in the amount of 3.8 g.

2) Synthesis of 1-trifluoromethylheptyl 4-hydroxybenzoate

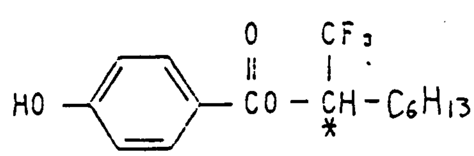

The compound obtained in 1) was dissolved in 100 ml of methanol, added with 10 % Pd/carbon catalyst and hydrogenated in hydrogen atmosphere to obtain the captioned compound in the amount of 2.8 g.

3) Synthesis of 4'-(1-trifluoromethylheptyloxycarbonyl)-phenyl 4-(5-n-nonyl-trans-1,3-dioxane-2-yl) benzoate

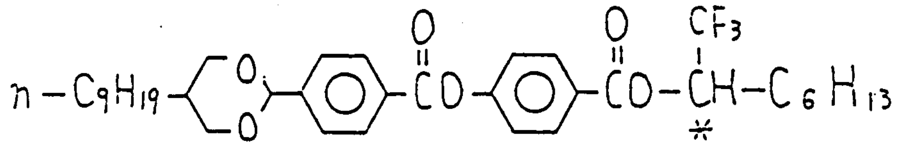

4-(5-Nonyl-trans-1,3-dioxane-2-yl)benzonitrile obtained according to the usual method was subjected to alkaline hydrolysis in ethylene glycol as solvent to obtain 4-(5-nonyl-trans-1,3-dioxy-2-yl)benzoic acid. This benzoic acid derivative in the amount of 0.700 g, 0.537 g of 1-trifluoromethylheptyl 4-hydroxybenzoate obtained in 2), 0.546 g of dicyclohexylcarbodiimide and a very small amount of dimethylaminopyridine were reacted in 30 ml of tetrahydrofuran at the room temperature for a whole day and night. The reaction mixture was filtered to remove the unsolved content and distilled for remove tetrahydrofuran. The residue was subjected to silica gel chromatography and recrystalized with ethanol to obtain the optically active objective compound in the amount of 0.179 g. The optical rotation thereof $[\alpha]_{20}^{D}$ = -30.47°.

This objective liquid crystal compound shows tristable liquid crystal phase and has following phase transition points as a result of observation by means of the polarizing microscope with a hot stage.

$$Cry \underset{18.0°C}{\overset{52.5°C}{\rightleftarrows}} S_{(3)}^{*} \underset{70.0°C}{\overset{71.0°C}{\rightleftarrows}} S_{C}^{*} \underset{71.5°C}{\overset{72.0°C}{\rightleftarrows}} S_{A} \underset{79.9°C}{\overset{80.0°C}{\rightleftarrows}} Iso$$

$S_{(s)}^{*}$ means the tristable liquid crystal phase.

The infrared absorption spectrum (KBr) thereof and the nuclear magnetic resonance spectrum are respectively shown in Fig. 2 and Table 1 given hereafter.

Example 3

1) Synthesis of (R)-(+)-1,1,1-trifluoromethyl-2-ethoxycarbonylethyl 4-benzyloxybenzoate

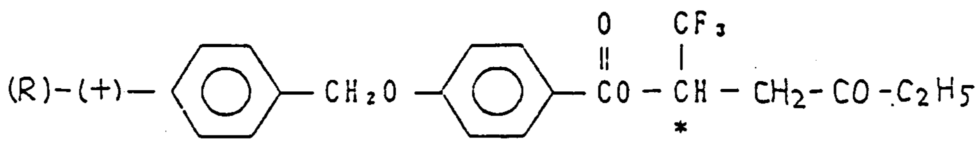

4-Benzyloxybenzoyl chloride in the amount of 1,8 g was dissolved in 30 ml of methylene chloride to prepare a solution, to which a solution of 1,2g of (R)-(+)-ethyl-4,4,4-trifluoro-2- hydroxybutylate ($[\alpha]_d$ = +21.80), 0.7 g of triethylamine and 0.2 g of dimethyl aminopyridine in 20 ml of methylene chloride was gradually added under cooling with ice, after which the reaction liquid was held at the room temperature to be stirred for 12 hours. The reaction liquid was poured into ice-water and extracted with methylene chloride. The methylene chloride phase was successively washed with diluted hydrochloric acid, water, 1N sodium carbonate solution and water so as to recover an organic phase, which was dried over anhydrous magnesium sulphate, distilled for removing the solvent and subjected to toluene/silica gel chromatography to obtain the captioned compound in the amount of 1.1 g.

2) Synthesis of (R)-(+)-1-trifluoromethyl-2-ethoxycarbonylethyl 4-hydroxybenzoate

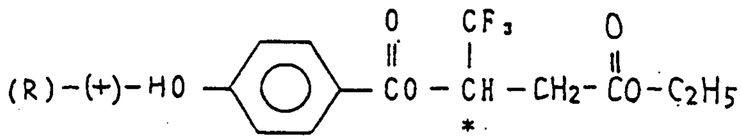

The compound obtained in 1) was dissolved in 50 ml of methanol, added with 10 % Pd/carbon catalyst and hydrogenated in hydrogene atmosphere to obtain the captioned compound in the amount of 0.8 g.

3) (R)-(+)-4-(1,1,1-trifluoromethyl-2-ethoxycarbonyloxycarbonyl)-phenyl 4-(5-n-pentyl-trans-1,3-dioxane-2-yl)benzoate

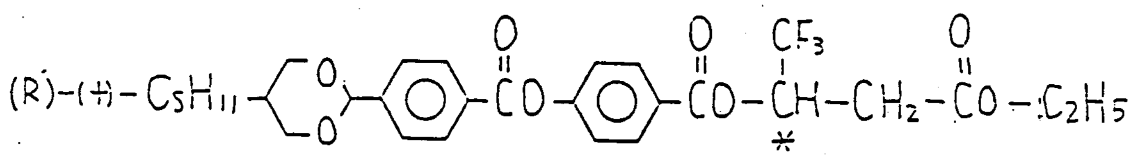

Separately, 1.2 g of 4-(5-pentyl-trans-1,3-dioxane-2-yl)benzonitrile prepared according to the usual method was subjected to alkaline hydrolysis with using ethylene glycol as solvent to obtain 4-(5-n-pentyl-trans-1,3-dioxane-2-yl)benzoic acid, which was reacted with thionyl in the exessive amount under reflux for 6 hours and distilled for removing unreacted thionyl to obtain the acid chloride in the amount of 0.9 g.

0.8 g of (R)-(+)-1,1,1-trifluoromethyl-2-ethoxycarbonylethyl 4-hydroxybenzoate obtained in 2), 0.3 g of triethylamine and 0.1 g of dimethylaminopyridine were dissolved in 50 ml of methylene chloride for preparing a solution, which was added to a solution of the acid chloride obtained in the above in 20 ml of methylene chloride for reaction at the room temperature for a whole day and might.

The reaction liquid was poured into ice-water and extracted with methylene chloride. The methylene chloride phase was successively washed with diluted hydrochloric acid, water, 1N sodium carbonate solution and water. The organic phase was dried over anhydrous sodium sulphate and distilled for separating the crude compound.

The crude compound was subjected to silica gel chromatography with using hexane/ethylacetate as developer so as to obtain purified objective compound ($[\alpha]_{20}^{D}$ = +24.5°) in the amount of 0.5 g.

The transition phase points thereof is as follows as a result of the observation by means of a polarizing microscope with a hot stage.

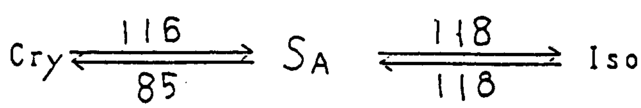


The infrared absorption spectrum (KBr) is shown in Fig. 3.

Example 4

In a liquid crystal cell casing of 2.3 µm thickness having an indium/tinoxide substrate coated with an orientated polyimide layer, the liquid crystal compounds obtained in Example 3 was filled in the isotropic phase to prepare a liquid crystal cell.

The cell was gradually cooled by a temperature gradient of 0.1 - 1.0°C/min so that the liquid crystal was orientated at the $S_A$ phase, impressed with a wave of ±15C and 10 Hz and observed by a polarizing microscope having a photomuliplier so as to detect the electrooptical respouse behavier, whereby the electroclinic effect making optical response to the impressed electric field at the $S_A$ phase was confirmed as

shown in Fig. 4.

The same can be applied also in respect of the liquid crystal compound obtained in Example 1.

Example 5

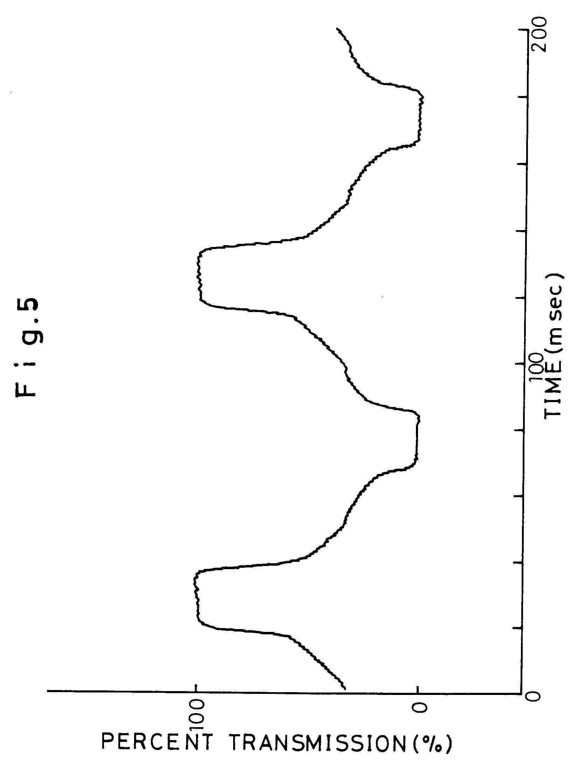

The similar liquid crystal cell except that the liquid crystal obtained in Example 2 was used instead of that of Example 3 was mounted in the polarizing microscope having the photomultiplier of two polarizing plates so arranged as to make a right angle with each other in such a way that the molecular apsis makes an angle of 22.5° relative to the polarizer. This liquid crystal cell was gradually cooled at a temperature gradient of 0.1 - 1.0°C/min to be in the $S_C^*$ phase. In the range or a temperature of 70.0°C to 18.0°C, a triangular wave voltage of ±30V, 10 Hz was impressed, of which state is shown in Fig. 5, from which it is possible to observe tristable liquid crystal molecular orientations comprising a dark phase in case of minus impressed voltage, a medium phase in case or zero voltage and a light phase in case of the plus voltage.

TABLE 1

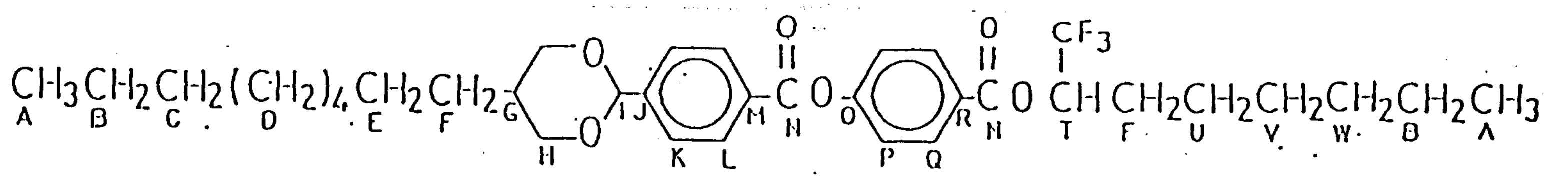

| Carbon | ppm | Remarks | Carbon | ppm | Remarks |
|---|---|---|---|---|---|
| A | 14.0 | Methyl Carbon | O | 155.2 | Aromatic Carbon Adjacent to O |
| B | 22.5 | Methylene Carbon | P | 122.0 | Aromatic Ring Carbon |
| C | 31.9 | " | Q | 131.6 | " |
| D | 29.5 | " | R | 126.4 | Aromatic Ring Carbon Adjacent to Carbonyl |
| E | 26.4 | " | S | 124.0 | $F_3$ Substituted Methyl Carbon |
| F | 28.2 | " | T | 70.3 | Methine Carbon Adjacent to $F_3$ Substituted Methyl |
| G | 34.2 | Methine Carbon | U | 24.6 | Methyl Carbon |
| H | 72.6 | Methylene Carbon Adjacent to O | V | 28.8 | " |
| I | 100.5 | Methine Carbon Adjacent to O | W | 31.5 | " |
| J | 144.3 | Aromatic Ring Carbon | | | |
| K | 126.5 | " | | | |
| L | 130.1 | " | | | |
| M | 129.2 | Aromatic Ring Carbon Adjacent to Carbonyl | | | |
| N | 164.1 | Carbonyl Carbon | | | |

## Claims

1. Liquid crystal compounds represented by the general formula (I)

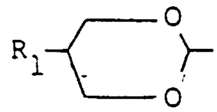 (A) - X - (B) - Y - $R_2$ (I)

in which $R_1$ means an alkyl, alkoxy, alkyloxycarbonyl or alkylcarbonyloxy group or 1 - 20 carbon atoms;
(A) means

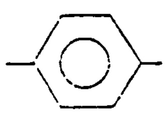 , 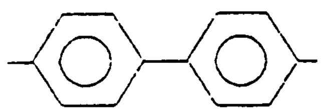 , 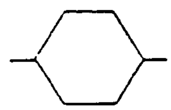 ,

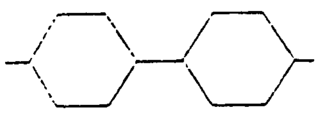 ; 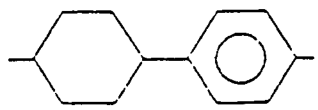 or

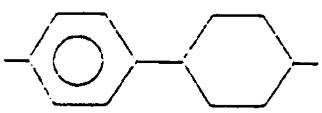

X means

$-\overset{O}{\overset{\|}{C}}O-$, $-O\overset{O}{\overset{\|}{C}}-$, $-(\overset{R'}{CH})_{\ell}-O-$ or $-O-(\overset{R'}{CH})_{\ell}-$,

wherein R' is hydrogen, methyl or ethyl and $\ell$ is an integer of 1 - 16;
(B) means

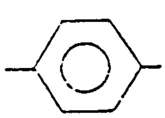 , 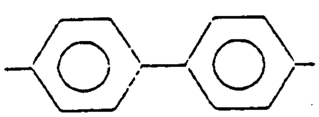 , 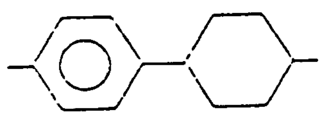 or

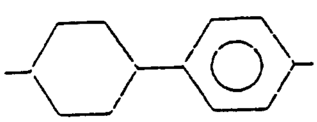

Y means

$-\overset{O}{\overset{\|}{C}}O-$,

-O-, -S- or $-CH_2O-$; and
$R_2$ means

$$-(CH_2)m-\overset{Z}{C}H-R_3 \quad \text{or} \quad -(CH_2)m-\overset{O}{\overset{\|}{C}}H-CH_2-\overset{O}{\overset{\|}{C}}O-R_3,$$

wherein $R_3$ is a straight or branched alkyl, aralkyl, alkoxyalkyl, alkoxycarbonyl or alkoxycarbonylalkyl group of 1 - 20 carbon atoms, a substituted vinyl, substituted cyclopropyl or substituted epoxy group; Z is $CF_3$, $C_2F_5$, $CHF_2$, $CH_2F$, $CClF_2$, $CCL_2F$, $CF_3CCl_2$ or $C_3F_7$; and m is an integer of 0 - 20.

2. Liquid crystal compounds as set forth in Claim 1, in the formula (I) of which $R_2$ means a radical derived from any of optically active alcohols;
1,1,1-trifluoro-2-$C_6$ - $C_{16}$ alkanol,
1,1-difluoro-2-$C_6$ - $C_{16}$ alkanol,
1-monofluoro-2-$C_6$ - $C_{16}$ alkanol,
1,1,1,2,2-pentafluoro-3-$C_6$ - $C_{16}$ alkanol,
1-monofluoro-1,1-dichloro-2-$C_6$ - $C_{16}$ alkanol,
1,1,1-trichloro-2-$C_6$ - $C_{16}$ alkanol,
1,1-difluoro-1-monochloro-2-$C_6$ - $C_{16}$ alkanol,
1,1,1-trifluoromethyl-1-phenylmethanol,
1,1,1-trifluoromethyl-2-phenylethanol,
1,1,1-trifluoromethyl-3-phenylpropanol,
1,1,1-trifluoro-3-decene-2-al,
1,1,1-trifluoro-3-heptene-2-al,
methyl-4,4,4-trifluoro-3-hydroxybutylate,
ethyl-4,4,4-trifluoro-3-hydroxybutylate,
propyl-4,4,4-trifluoro-3-hydroxybutylate,
butyl-4,4,4-trifluoro-3-hydroxybutylate,
pentyl-4,4,4-trifluoro-3-hydroxybutylate,
hexyl-4,4,4-trifluoro-3-hydroxybutylate,
octyl-4,4,4-trifluoro-3-hydroxybutylate,
nonyl-4,4,4-trifluoro-3-hydroxybutylate and
decyl-4,4,4-trifluoro-3-hydroxybutylate.

3. Liquid crystal compounds as set forth in Claim 1, in the formula (I) of which $R_1$ means an alkylo, alkoxy, alkyloxycarbonyl or alkylcarbonyloxy group of 4 - 20 carbon atoms;
(A) means

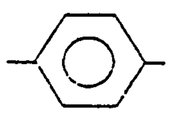, 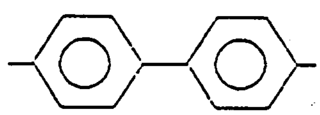, 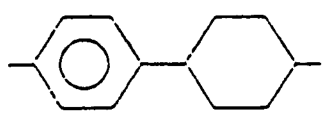 or

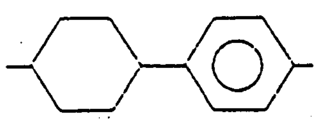

X means

$$-\overset{O}{\overset{\|}{C}}O-, \quad -O\overset{O}{\overset{\|}{C}}-,$$

$CH_2O$, -O- or -$OCH_2$;

(B) means

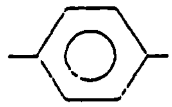 or 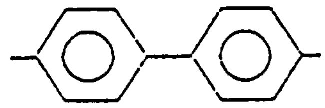

Y means

$$-\overset{O}{\overset{\|}{C}}O-,$$

-O-, -S- or $-CH_2O-$; and
$R_2$ means

$$-(CH_2)m-\overset{Z}{C}H-R_3 \text{ or } -(CH_2)m-\overset{Z}{C}H-CH_2-\overset{O}{\overset{\|}{C}}O-R_3$$

wherein $R^3$ is a straight or branched alkyl group or 3 - 15 carbon atoms; Z is $CH_3$, $C_2F_5$, $CHF_2$, $CClF_2$, $CCL_2F$, $CF_3CCL_2$ or $C_3F_7$; and m is an integer of 0 - 15.

**4.** Liquid crystal compounds as set forth in Claim 1, in the formula (I) of which $R_1$ means $C_nH_{2n+1}$, $C_nH_{2n+1}O$,

$$C_nH_{2n+1}O\overset{O}{\overset{\|}{C}} \text{ or } C_nH_{2n+1}\overset{O}{\overset{\|}{C}}O,$$

wherein n is an integer of 5 - 10;
(A) means

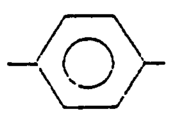, 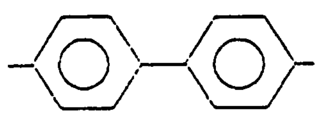, 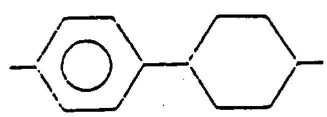 or

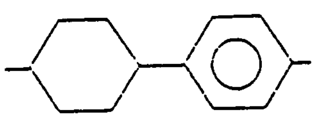

X means

$$-\overset{O}{\overset{\|}{C}}O- \text{ or } -O\overset{O}{\overset{\|}{C}}-;$$

(B) means

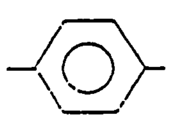 or 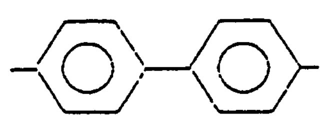

Y means

$$-\overset{O}{\overset{\|}{C}}O-,$$

-O-, -S- or $-CH_2O-$;
and $R_2$ means

$$-(CH_2)m-\overset{Z}{\overset{|}{C}}H-C_nH_{2n+1} \text{ or } -(CH_2)m-\overset{Z}{\overset{|}{C}}H-CH_2-\overset{O}{\overset{\|}{C}}O-R_3$$

wherein $R_3$ is a straight or branched alkyl broup of 3 - 15 carbon atoms; Z is $CF_3$, $C_2F_5$ or $C_3F_7$; m is an integer of 0 - 10 and n is an integer of 5 - 10.

**5.** Liquid crystal compounds as set forth in Claim 2, in the formula (I) of which $R^2$ means the radical derived from any of optically active alcohols;
1,1,1-trifluoro-2-$C_6$ - $C_{16}$ alkanol,
1,1-difluoro-2-$C_6$ - $C_{16}$ alkanol,
1-monofluoro-2-$C_6$ - $C_{16}$ alkanol,
1,1,1,2,2-pentafluoro-3-$C_6$ - $C_{16}$ alkanol,
1-monofluoro-1,1-dichloro-2-$C_6$ - $C_{16}$ alkanol,
1,1,1-trichloro-2-$C_6$ - $C_{16}$ alkanol and
1,1-diifluoro-1-monochloro-2-$C_6$ - $C_{16}$ alkanol.

**6.** Liquid crystal compounds as set forth in Claim 1, in the formula (I) of which $R_1$ means an alkyl, alkoxy, alkyloxycarbonyl or alkylcarbonyloxy group of 5 - 20 carbon atoms;
(A) means

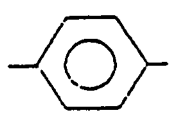 or 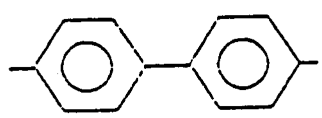

X means

$$-\overset{O}{C}O- \text{ or } -O\overset{O}{C}-;$$

(B) means

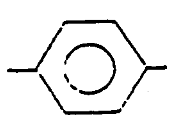 or 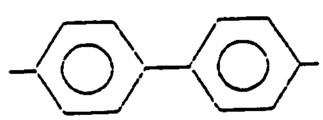

Y means

$$-\overset{O}{\overset{\|}{C}}O-;$$

and
$R_2$ means

$$-(CH_2)m-\overset{Z}{CH}-R_3$$

wherein $R^3$ is a straight or branched alkyl group of 3 - 15 carbon atoms, Z is $CF_3$, $C_2F_5$, $CHF_2$, $CH_2F$, $CClF_2$, $CCl_2F$ or $CF_3CCl_2$; and m is an integer of 0 - 3, so as to show a tristable liquid crystal phase.

**7.** Liquid crystal compounds as set forth in Claim 6, in which, $R_1$ means a straight chain alkyl or alkoxy group respectively represented by the formula, $C_nH_{2n+1}$ or $C_nH_{2n+1}O$ wherein n is an integer or 6 - 10;
(A) means

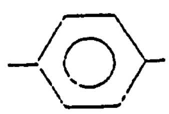 or 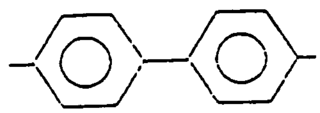

X means

$$-\overset{O}{\overset{\|}{C}}O- \text{ or } -O\overset{O}{\overset{\|}{C}}-;$$

(B) means

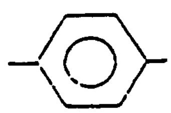 or 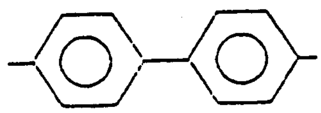

Y means

$$-\overset{O}{\overset{\|}{C}}O-;$$

and
$R_2$ means

$$-(CH_2)m-\overset{Z}{CH}-C_nH_{2n+1},$$

wherein Z is $CF_3$ or $C_2F_5$, m is an integer of 0 or 1 and n is an integer of 5, 6, 8, 9, or 10, so as to show a tristable liquid crystal phase.

**8.** Liquid crystal compounds as set forth in Claim 6, in which $R_2$ means a radical derived from any of optically active alcohols;
1,1,1-trifluoro-2-$C_6$ - $C_{16}$ alkanol,
1,1-difluoro-2-$C_6$ - $C_{16}$ alkanol,
1-monofluoro-2-$C_6$ - $C_{16}$ alkanol,
1,1,1,2,2-pentafluoro-3-$C_6$ - $C_{16}$ alkanol,
1-monofluoro-1,1-dichloro-2-$C_6$ - $C_{16}$ alkanol,
1,1,1-trichloro-2-$C_6$ - $C_{16}$ alkanol and
1,1-diifluoro-1-monochloro-2-$C_6$ - $C_{16}$ alkanol.

9. Liquid crystal compound as set forth in Claim 1, which is (R)-( + )-4'-(1-trifluoromethylnonyloxycarbonyl)-phenyl 4-(5-pentyl-trans-1,3-dioxane-2-yl)benzoate of the formula;

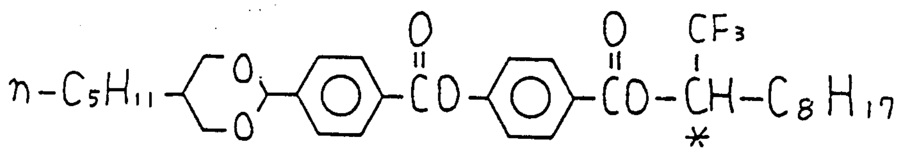

10. Liquid crystal compound as set forth in Claim 1, which is 4'-(1-trifluoromethylheptyloxycarbonyl)phenyl 4-(5-n-nonyl-trans-1,3-dioxane-2-yl)-benzoate showing a tristable liquid crystal phase and of the formula;

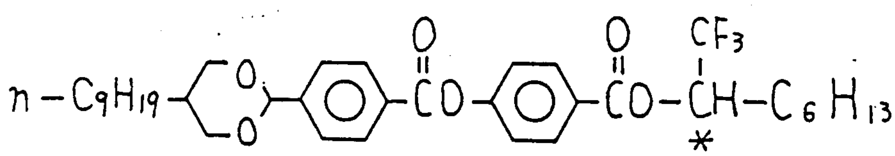

11. Liquid crystal compound as set forth in Claim 1, which is (R)-( + )-4-(1-trifluoromethyl-2-ethoxycarbonyloxycarbonyl)-phenyl 4-(5-n-pentyl-trans-1,3-dioxane-2-yl) benzoate of the formula;

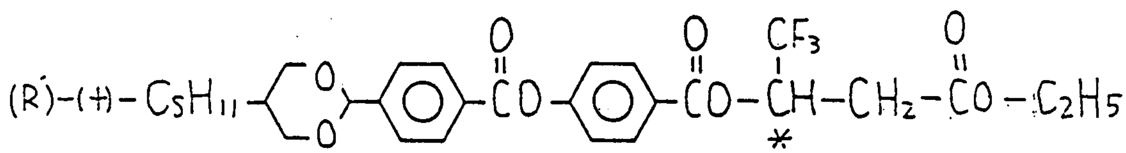

**Patentansprüche**

1. Flüssigkristall-Verbindungen, die durch die allgemeine Formel (I)

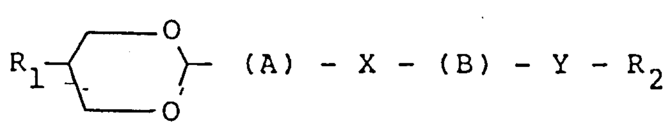

(I)

repräsentiert werden, worin $R_1$ eine Alkyl-, Alkoxy-, Alkyloxycarbonyl- oder Alkylcarbonyloxy-Gruppe von 1 - 20 Kohlenstoffatomen bedeutet;

(A)

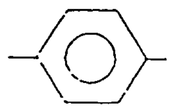, 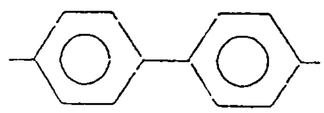, 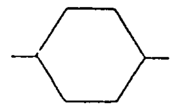,

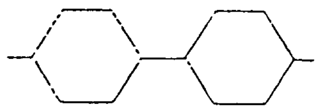; 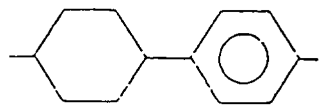 oder

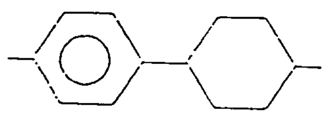

bedeutet
X

$-\overset{\overset{O}{\|}}{C}O-$, $-O\overset{\overset{O}{\|}}{C}-$, $-(\overset{\overset{R'}{|}}{C}H)_l-O-$ oder $-O-(\overset{\overset{R'}{|}}{C}H)_l-$

bedeutet, worin R' Wasserstoff, Methyl oder Ethyl ist und l eine ganze Zahl von 1 - 16 ist;
(B)

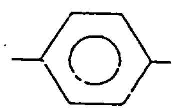, 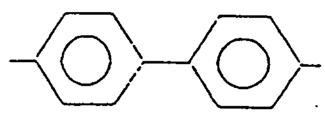, 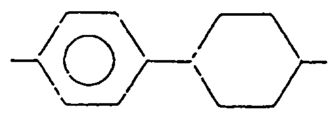.

oder 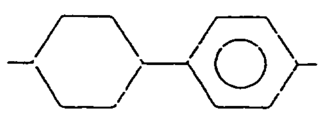

bedeutet,
Y

$-\overset{\overset{O}{\|}}{C}O-$,

-O-, -S- oder $-CH_2O-$ bedeutet; und
$R_2$

$-(CH_2)m-\overset{\overset{Z}{|}}{C}H-R_3$ oder $-(CH_2)m-\overset{\overset{O}{\|}}{C}H-CH_2-\overset{\overset{O}{\|}}{C}O-R_3$

bedeutet, worin $R_3$ eine geradkettige oder verzweigte Alkyl-, Aralkyl-, Alkoxyalkyl-, Alkoxycarbonyl- oder Alkoxycarbonylalkyl-Gruppe von 1 - 20 Kohlenstoffatomen, eine substituierte Vinyl-, substituierte Cyclopropyl- oder substituierte Epoxygruppe ist; Z $CF_3$, $C_2F_5$, $CHF_2$, $CH_2F$, $CClF_2$, $CCl_2F$, $CF_3CCl_2$ oder $C_3F_7$ ist; und <u>m</u> eine ganze Zahl von 0 - 20 ist.

2. Flüssigkristall-Verbindungen nach Anspruch 1, worin $R_2$ in Formel (I) einen von irgendeinem der optisch aktiven Alkohole abgeleiteten Rest bedeutet;
1,1,1-Trifluor-2-$C_6$ - $C_{16}$-Alkanol,
1,1-Difluor-2-$C_6$ - $C_{16}$-Alkanol,
1-Monofluor-2-$C_6$ - $C_{16}$-Alkanol,
1,1,1,2,2-Pentafluor-3-$C_6$ - $C_{16}$-Alkanol,
1-Monofluor-1,1-dichlor-2-$C_6$ - $C_{16}$-Alkanol,
1,1,1-Trichlor-2-$C_6$ - $C_{16}$-Alkanol,
1,1-Difluor-1-monochlor-2-$C_6$ - $C_{16}$-Alkanol,
1,1,1-Trifluormethyl-1-phenylmethanol,
1,1,1-Trifluormethyl-2-phenylethanol,
1,1,1-Trifluormethyl-3-phenylpropanol,
1,1,1-Trifluor-3-decen-2-al,
1,1,1-Trifluor-3-hepten-2-al,
Methyl-4,4,4-trifluor-3-hydroxybutylat,
Ethyl-4,4,4-trifluor-3-hydroxybutylat,
Propyl-4,4,4-trifluor-3-hydroxybutylat,
Butyl-4,4,4-trifluor-3-hydroxybutylat,
Pentyl-4,4,4-trifluor-3-hydroxybutylat,
Hexyl-4,4,4-trifluor-3-hydroxybutylat,
Octyl-4,4,4-trifluor-3-hydroxybutylat,
Nonyl-4,4,4-trifluor-3-hydroxybutylat und
Decyl-4,4,4-trifluor-3-hydroxybutylat.

3. Flüssigkristall-Verbindungen nach Anspruch 1, worin $R_1$ in Formel (I) eine Alkyl-, Alkoxy-, Alkyloxycarbonyl- oder Alkylcarbonyloxy-Gruppe von 4 - 20 Kohlenstoffatomen bedeutet;
(A)

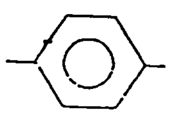 , 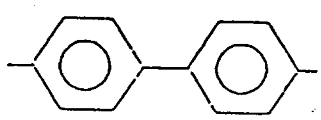 , 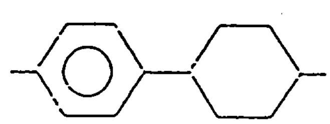 oder

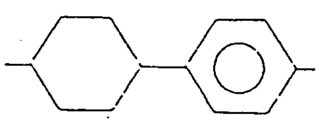

bedeutet,
X

$-\overset{\overset{O}{\|}}{C}O-$, $-O\overset{\overset{O}{\|}}{C}-$,

$CH_2O$, -O- oder $-OCH_2$ bedeutet;

(B)

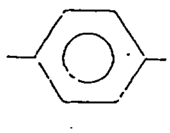

oder

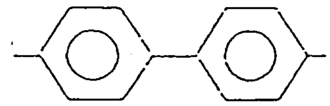

bedeutet;

Y

$$-\overset{O}{\overset{\|}{C}}O-,$$

-O-, -S- oder $-CH_2O-$ bedeutet; und

$R_2$

$$-(CH_2)m-\overset{Z}{\overset{|}{C}}H-R_3 \text{ oder } -(CH_2)m-\overset{Z}{\overset{|}{C}}H-CH_2-\overset{O}{\overset{\|}{C}}O-R_3$$

bedeutet, worin $R_3$ eine geradkettige oder verzweigte Alkylgruppe mit 3 - 15 Kohlenstoffatomen ist; Z $CH_3$, $C_2F_5$, $CHF_2$, $CClF_2$, $CCl_2F$, $CF_3CCl_2$ oder $C_3F_7$ ist; und m eine ganze Zahl von 0 - 15 ist.

4. Flüssigkristall-Verbindungen nach Anspruch 1, worin $R_1$ in Formel (I) $C_nH_{2n+1}$, $C_nH_{2n+1}O$,

$$C_nH_{2n+1}O\overset{O}{\overset{\|}{C}} \text{ oder } C_nH_{2n+1}\overset{O}{\overset{\|}{C}}O$$

bedeutet, worin n eine ganze Zahl von 5 - 10 ist;

(A)

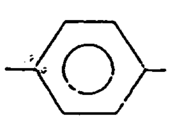 , 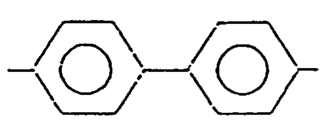 , 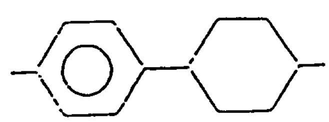

oder

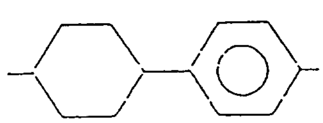

bedeutet,

X

$$-\overset{O}{\overset{\|}{C}}O- \text{ oder } -O\overset{O}{\overset{\|}{C}}-$$

bedeutet;
(B)

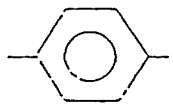 oder 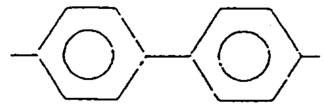

bedeutet,
Y

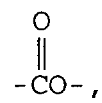

-O-, -S- oder $-CH_2O-$ bedeutet; und
$R_2$

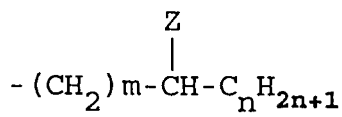 oder 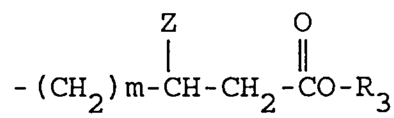

bedeutet, worin $R_3$ eine geradkettige oder verzweigte Alkylgruppe von 3 - 15 Kohlenstoffatomen ist; Z $CF_3$, $C_2F_5$ oder $C_3F_7$ ist; m eine ganze Zahl von 0 - 10 ist und n eine ganze Zahl von 5 - 10 ist.

5. Flüssigkristall-Verbindungen nach Anspruch 2, worin $R_2$ in Formel (I) den von irgendeinem der optisch aktiven Alkohole abgeleiteten Rest bedeutet;
1,1,1-Trifluor-2-$C_6$ - $C_{16}$ Alkanol,
1,1-Difluor-2-$C_6$ - $C_{16}$ Alkanol,
1-Monofluor-2-$C_6$ - $C_{16}$ Alkanol,
1,1,1,2,2-Pentafluor-3-$C_6$ - $C_{16}$ Alkanol,
1-Monofluor-1,1-dichlor-2-$C_6$ - $C_{16}$ Alkanol,
1,1,1-Trichlor-2-$C_6$ - $C_{16}$ Alkanol, und
1,1-Difluor-1-monochlor-2-$C_6$ - $C_{16}$ Alkanol.

6. Flüssigkristall-Verbindungen nach Anspruch 1, worin $R_1$ in Formel (I) eine Alkyl-, Alkoxy-, Alkyloxycarbonyl- oder Alkylcarbonyloxy-Gruppe von 5 - 20 Kohlenstoffatomen bedeutet;
(A)

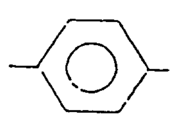 oder 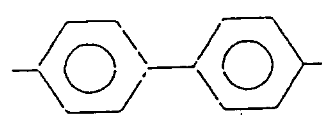

bedeutet,
X

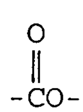 oder 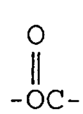

bedeutet;

(B)

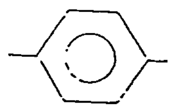 oder 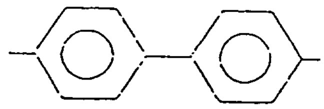

bedeutet,
Y

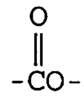

bedeutet; und
$R_2$

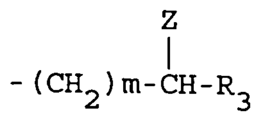

bedeutet, worin $R_3$ eine geradkettige oder verzweigte Alkylgruppe von 3 - 15 Kohlenstoffatomen ist, Z $CF_3$, $C_2F_5$, $CHF_2$, $CH_2F$, $CClF_2$, $CCl_2F$ oder $CF_3CCl_2$ist; und m eine ganze Zahl von 0 - 3 ist, um eine tristabile Flüssigkristallphase aufzuweisen.

7. Flüssigkristall-Verbindungen nach Anspruch 6, worin $R_1$ eine geradkettige Alkyl- oder Alkoxygruppe, repräsentiert durch die Formel $C_nH_{2n+1}$ oder $C_nH_{2n+1}O$, bedeutet, worin n eine ganze Zahl von 6 - 10 ist;
(A)

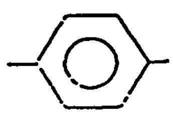 oder 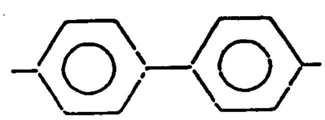

bedeutet,
X

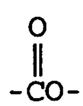 oder 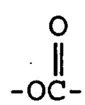

bedeutet;
(B)

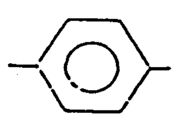 oder 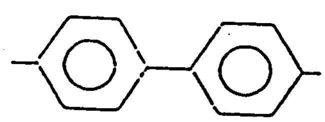

bedeutet,
Y

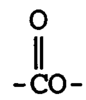

bedeutet; und
$R_2$

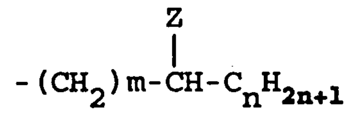

bedeutet, worin Z $CF_3$ oder $C_2F_5$ ist, m eine ganze Zahl von 0 oder 1 ist und n eine ganze Zahl von 5, 6, 8, 9 oder 10 ist, um eine tristabile Flüssigkristallphase aufzuweisen.

8. Flüssigkristall-Verbindungen nach Anspruch 6, worin $R_2$ einen von irgendeinem der optisch aktiven Alkohole abgeleiteten Rest bedeutet;
1,1,1-Trifluor-2-$C_6$ - $C_{16}$ Alkanol,
1,1-Difluor-2-$C_6$ - $C_{16}$ Alkanol,
1-Monofluor-2-$C_6$ - $C_{16}$ Alkanol,
1,1,1,2,2-Pentafluor-3-$C_6$ - $C_{16}$ Alkanol,
1-Monofluor-1,1-dichlor-2-$C_6$ - $C_{16}$ Alkanol,
1,1,1-Trichlor-2-$C_6$ - $C_{16}$ Alkanol, und
1,1-Difluor-1-monochlor-2-$C_6$ - $C_{16}$ Alkanol.

9. Flüssigkristall-Verbindung nach Anspruch 1, die (R)-(+)-4'-(1-Trifluormethylnonyloxycarbonyl)-phenyl 4-(5-pentyl-trans-1,3-dioxan-2-yl)benzoat der Formel

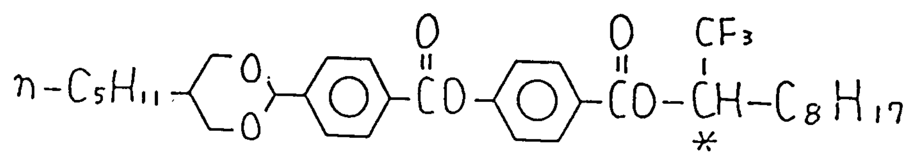

ist.

10. Flüssigkristall-Verbindung nach Anspruch 1, die 4'-(1-Trifluormethylheptyloxycarbonyl)-phenyl 4-(5-n-nonyl-trans-1,3-dioxan-2-yl)-benzoat ist, eine tristabile Flüssigkristallphase aufweist und die Formel

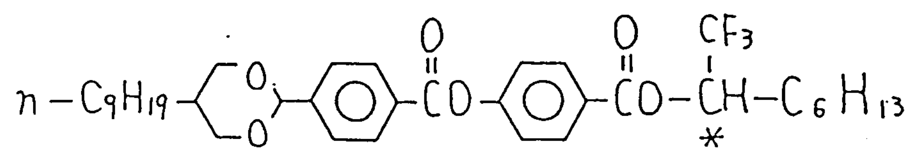

besitzt.

11. Flüssigkristall-Verbindung nach Anspruch 1, die (R)-(+)-4-(1-Trifluormethyl-2-ethoxycarbonyloxycarbonyl)phenyl 4-(5-n-pentyl-trans-1,3-dioxan-2-yl)-benzoat der Formel

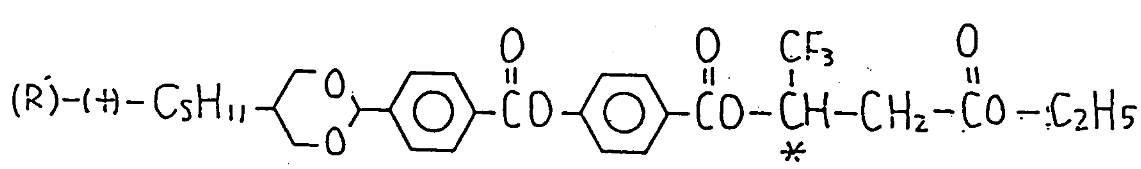

ist.

**Revendications**

**1.** Cristaux liquides représentés par la formule développée (I)

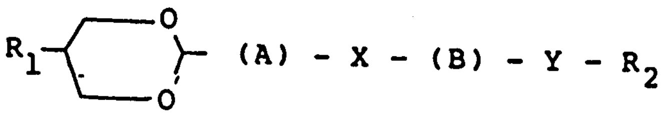

(I)

dans laquelle $R_1$ signifie un groupe alcoyle, alcoxy, alcoyloxycarbonyle ou alcoylcarbonyloxy de 1 à 20 atomes de carbone,

(A) signifie

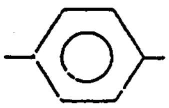,
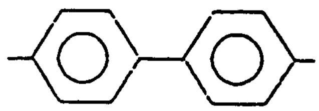,
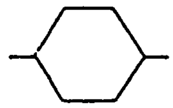,

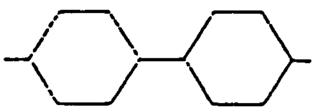,
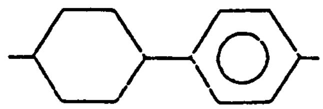 ou

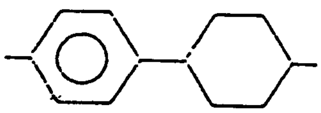

X signifie

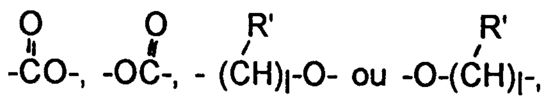

dans lesquels R' est hydrogène, méthyle ou éthyle et l est un nombre entier de 1 à 16,

(B) signifie

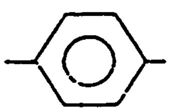,
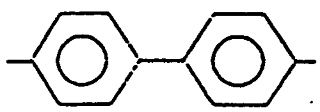,
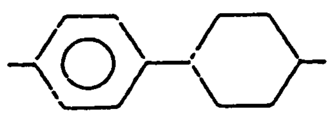.
ou

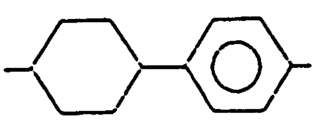

Y signifie

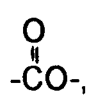

-O-, -S- ou $-CH_2O-$; et
$R_2$ signifie

$$-(CH_2)m-\overset{\overset{\Large Z}{|}}{C}H-R_3 \text{ ou } -(CH_2)m-\overset{\overset{\Large O}{||}}{C}H-CH_2-\overset{\overset{\Large O}{||}}{C}O-R_3,$$

dans lesquelles $R_3$ est un groupe alcoyle, aralcoyle, alcoxyalcoyle, alcoxycarbonyle ou alcoxycarbonylalcoyle linéaire ou ramifié de 1 à 20 atomes de carbone, un groupe vinyle substitué, cyclopropyle substitué ou époxy substitué, Z est $CF_3$, $C_2F_5$, $CHF_2$, $CH_2F$, $CClF_2$, $CCL_2F$, $CF_3CCl_2$ ou $C_3F_7$; et <u>m</u> est un nombre entier de 0 à 20.

2. Cristaux liquides suivant la revendication 1, dans la formule (I) desquels $R_2$ signifie un radical dérivé de l'un quelconque des alcools optiquement actifs;
1,1,1-trifluoro-2-alcanol ayant de 6 à 16 atomes de carbone,
1,1-difluoro-2-alcanol ayant de 6 à 16 atomes de carbone,
1-monofluoro-2-alcanol ayant de 6 à 16 atomes de carbone,
1,1,1,2,2-pentafluoro-3-alcanol ayant de 6 à 16 atomes de carbone,
1-monofluoro-1,1-dichloro-2-alcanol ayant de 6 à 16 atomes de carbone,
1,1,1-trichloro-2-alcanol ayant de 6 à 16 atomes de carbone,
1,1-difluoro-1-monochloro-2 ayant de 6 à 16 atomes de carbone,
1,1,1-trifluorométhyl-1-phénylméthanol,
1,1,1-trifluorométhyl-2-phényléthanol,
1,1,1-trifluorométhyl-3-phénylpropanol,
1,1,1-trifluoro-3-décène-2-al,
1,1,1-trifluoro-3-heptène-2-al,
4,4,4-trifluoro-3-hydroxybutylate de méthyle,
4,4,4-trifluoro-3-hydrobutylate d'éthyle,
4,4,4-trifluoro-3-hydrobutylate de propyle,
4,4,4-trifluoro-3-hydrobutylate de butyle,
4,4,4-trifluoro-3-hydrobutylate de pentyle,
4,4,4-trifluoro-3-hydrobutylate d'hexyle,
4,4,4-trifluoro-3-hydrobutylate d'octyle,
4,4,4-trifluoro-3-hydrobutylate de nonyle, et
4,4,4-trifluoro-3-hydrobutylate de décyle.

3. Cristaux liquides suivant la revendication 1, dans la formule (I) desquels $R_1$ signifie un groupe alcoyle, alcoxy, alcoyloxycarbonyle ou alcoylcarbonyloxy de 4 à 20 atomes de carbone;
(A) signifie

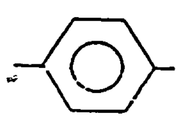 , 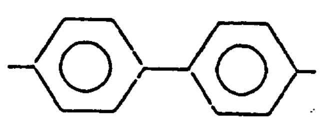 , 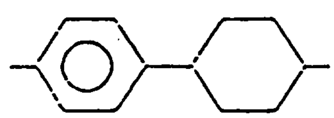 ou

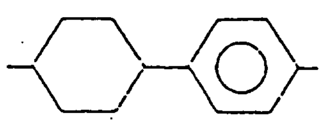

X signifie

$$-\overset{\overset{\Large O}{||}}{C}O-, \; -O\overset{\overset{\Large O}{||}}{C}-,$$

$CH_2O$, -O- ou $-OCH_2$;
(B) signifie

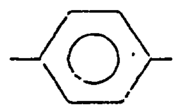 ou 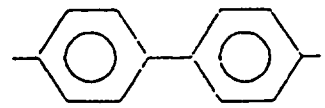

Y signifie

$$-\overset{O}{\overset{\|}{C}}O-,$$

-O-, -S- ou $-CH_2O-$; et
$R_2$ signifie

$$-(CH_2)m-\overset{Z}{\overset{|}{C}}H-R_3 \text{ ou } -(CH_2)m-\overset{Z}{\overset{|}{C}}H-CH_2-\overset{O}{\overset{\|}{C}}O-R_3$$

dans lesquels $R_3$ est un groupe alcoyle linéaire ou ramifié de 3 à 15 atomes de carbone, Z est $CH_3$, $C_2F_5$, $CHF_2$, $CClF_2$, $CCL_2F$, $CF_3CCl_2$ ou $C_3F_7$; et m est un nombre entier de 0 à 15.

**4.** Cristaux liquides suivant la revendication 1 dans la formule (I) desquels $R_1$ signifie $C_nH_{2n+1}$,

$$C_nH_{2n+1}O,\ \overset{O}{\overset{|}{C}}_nH_{2n+1}O\overset{O}{\overset{\|}{C}}$$

ou $C_nH_{2n+1}CO$ n étant un nombre entier de 5 à 10;
(A) signifie

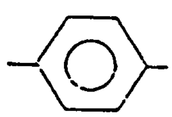, 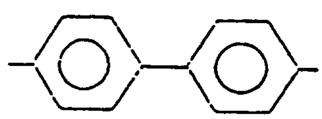, 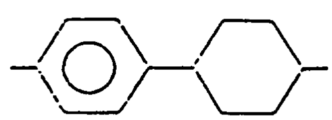 ou

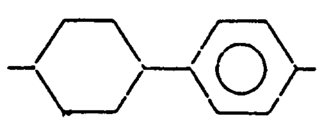

X signifie

$$-\overset{O}{\overset{\|}{C}}O- \text{ ou } -O\overset{O}{\overset{\|}{C}}-;$$

(B) signifie

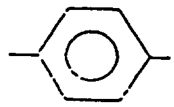 ou 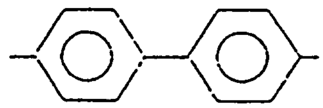

Y signifie

$$-\overset{O}{\overset{\|}{C}}O-,$$

-O-, -S- ou $-CH_2O-$;
et $R_2$ signifie

$$-(CH_2)m-\overset{Z}{\overset{|}{C}}H-C_nH_{2n+1} \text{ ou } -(CH_2)m-\overset{Z}{\overset{|}{C}}H-CH_2-\overset{O}{\overset{\|}{C}}O-R_3$$

dans lesquels $R_3$ est un groupe alcoyle linéaire ou ramifie de 3 à 15 atomes de carbone; Z est $CF_3$, $C_2F_5$ ou $C_3F_7$; m est un nombre entier de 0 à 10 et n est un nombre entier de 5 à 10.

**5.** Cristaux liquides suivant la revendication 2, dans la formule (I) desquels $R^2$ signifie le radical dérivé de l'un des alcools optiquement actifs suivants :
1,1,1-trifluoro-2-alcanol ayant de 6 à 16 atomes de carbone,
1,1-difluoro-2-alcanol ayant de 6 à 16 atomes de carbone,
1-monofluoro-2-alcanol ayant de 6 à 16 atomes de carbone,
1,1,1,2,2-pentafluoro-3-alcanol ayant de 6 à 16 atomes de carbone,
1-monofluoro-1,1-dichloro-2-alcanol ayant de 6 à 16 atomes de carbone,
1,1,1-trichloro-2-alcanol ayant de 6 à 16 atomes de carbone et
1,1-difluoro-1-monochloro-2-alcanol ayant de 6 à 16 atomes de carbone.

**6.** Cristaux liquides suivant la revendication 1, dans la formule (I) desquels $R_1$ signifie un groupe alcoyle, alcoxy, alcoylоxycarbonyle ou alcoylcarbonyloxy de 5 à 20 atomes de carbone;
(A) signifie

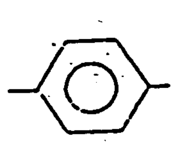 ou 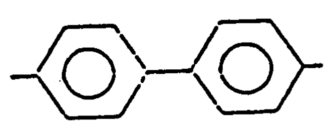

X signifie

$$-\overset{O}{\overset{\|}{C}}O- \text{ ou } -O\overset{O}{\overset{\|}{C}}-;$$

(B) signifie

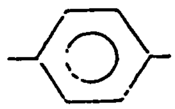 ou 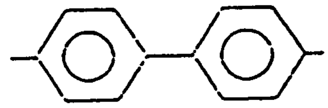

Y signifie

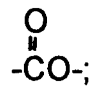

et
$R_2$ signifie

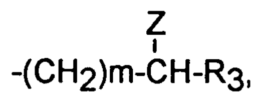

dans laquelle $R^3$ est un groupe alcoyle linéaire ou ramifié ayant de 3 à 4 atomes de carbone, Z est $CF_3$, $C_2F_5$, $CHF_2$, $CH_2F$, $CClF_2$, $CCl_2F$ ou $CF_3CCl_2$; et m est un nombre entier de 0 à 3 de manière à présenter une phase tristable de cristal liquide.

**7.** Cristaux liquides suivant la revendication 6, dans lesquels $R_1$ signifie un groupe alcoyle ou alcoxy linéaire représenté respectivement par la formule $C_nH_{2n+1}$ ou $C_nH_{2n+1}O$, n étant un nombre entier de 6 à 10;
(A) signifie

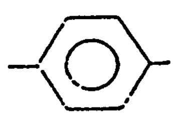 ou 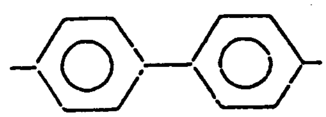

X signifie

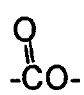 ou 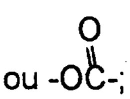;

(B) signifie

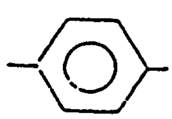 ou 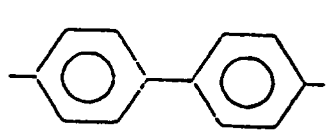

Y signifie

$$-\overset{\overset{\displaystyle O}{\|}}{C}O-;$$

et
$R_2$ signifie

$$-(CH_2)m-\overset{\overset{\displaystyle Z}{|}}{C}H-C_nH_{2n+1},$$

dans laquelle Z est $CF_3$ ou $C_2F_5$, m est un nombre entier égal à 0 ou à 1 et n est un nombre entier égal à 5, 6, 8, 9 ou 10, de manière à présenter une phase tristable de cristal liquide.

**8.** Cristaux liquides suivant la revendication 6, dans lesquels $R_2$ signifie un radical dérivé de l'un quelconque des alcools optiquement actifs suivants;
1,1,1-trifluoro-2-alcanol ayant de 6 à 16 atomes de carbone,
1,1-difluoro-2-alcanol ayant de 6 à 16 atomes de carbone,
1-monofluoro-C-alcanol ayant de 6 à 16 atomes de carbone
1,1,1,2,2-pentafluoro-2-alcanol ayant de 6 à 16 atomes de carbone,
1-monofluoro-1,1-dichloro-2-alcanol ayant de 6 à 16 atomes de carbone
1,1,1-trichloro-2-alcanol ayant de 6 à 16 atomes de carbone et
1,1-diifluoro-1-monochloro-2--alcanol ayant de 6 à 16 atomes de carbone.

**9.** Cristal liquide suivant la revendication 1, qui est le (R)-(+)-4-(5-pentyl-trans-1,3-dioxane-2-yl)benzoate de 4'-(1 -trifluorométhylnonyloxycarbonyl)phényle de formule;

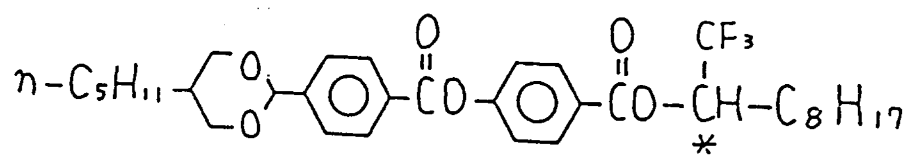

**10.** Cristal liquide suivant la revendication 1, qui est le 4-(5-n-nonyl-trans-1,3-dioxane-2-yl)benzoate de 4'-(1-trifluorométhylheptyloxycarbonyl)-phényle présentant une phase tristable de cristal liquide et répondant à la formule

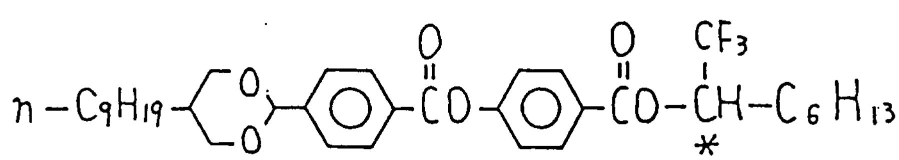

**11.** Cristal liquide suivant la revendication 1, qui est le (R)-(+)-4-(5-n-pentyl-trans-1,3-dioxane-2-yl)benzoate de 4-(1-trifluorométhyl-2-éthoxycarbonyloxycarbonyl)-phényle de formule;

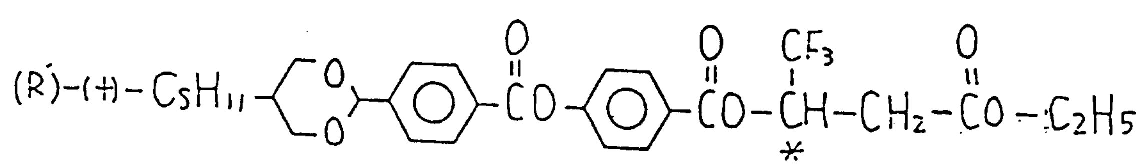

Fig.1

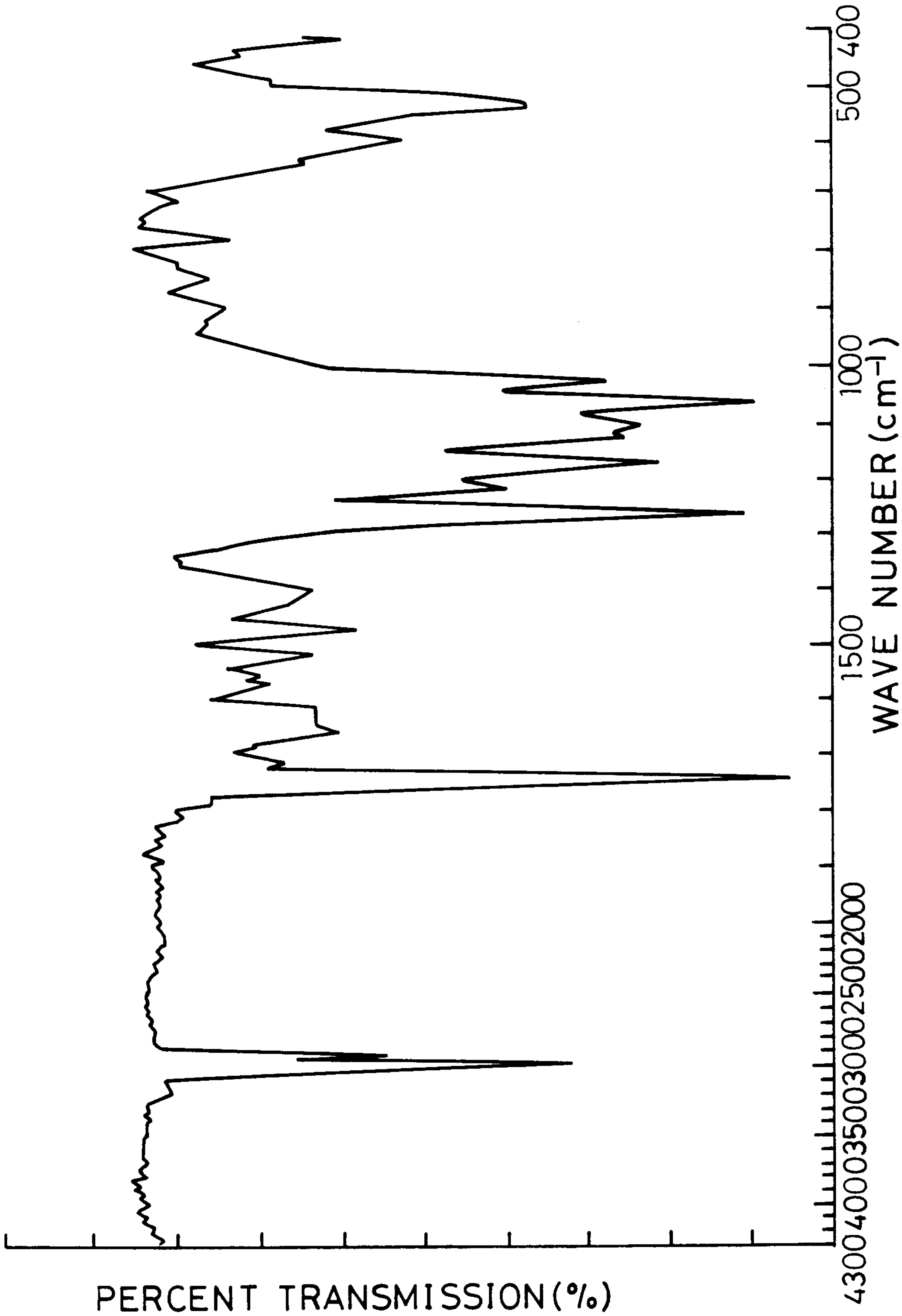
PERCENT TRANSMISSION(%)
WAVE NUMBER (cm$^{-1}$)
4300
4000
3500
3000
2500
2000
1500
1000
500
400

Fig. 2

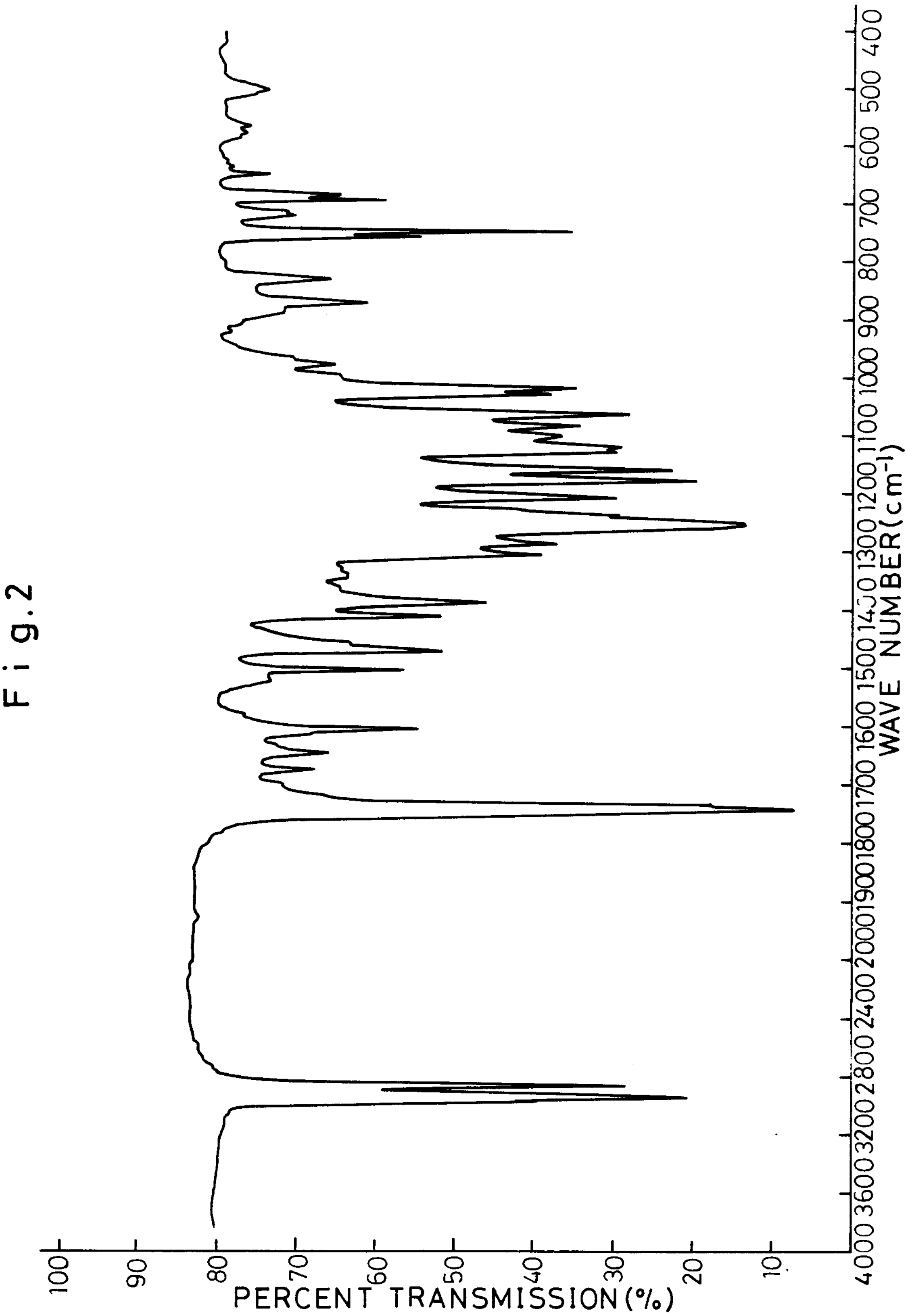
PERCENT TRANSMISSION (%)
100
90
80
70
60
50
40
30
20
10
WAVE NUMBER (cm⁻¹)
4000 3600 3200 2800 2400 2000 1900 1800 1700 1600 1500 1400 1300 1200 1100 1000 900 800 700 600 500 400

Fig.3

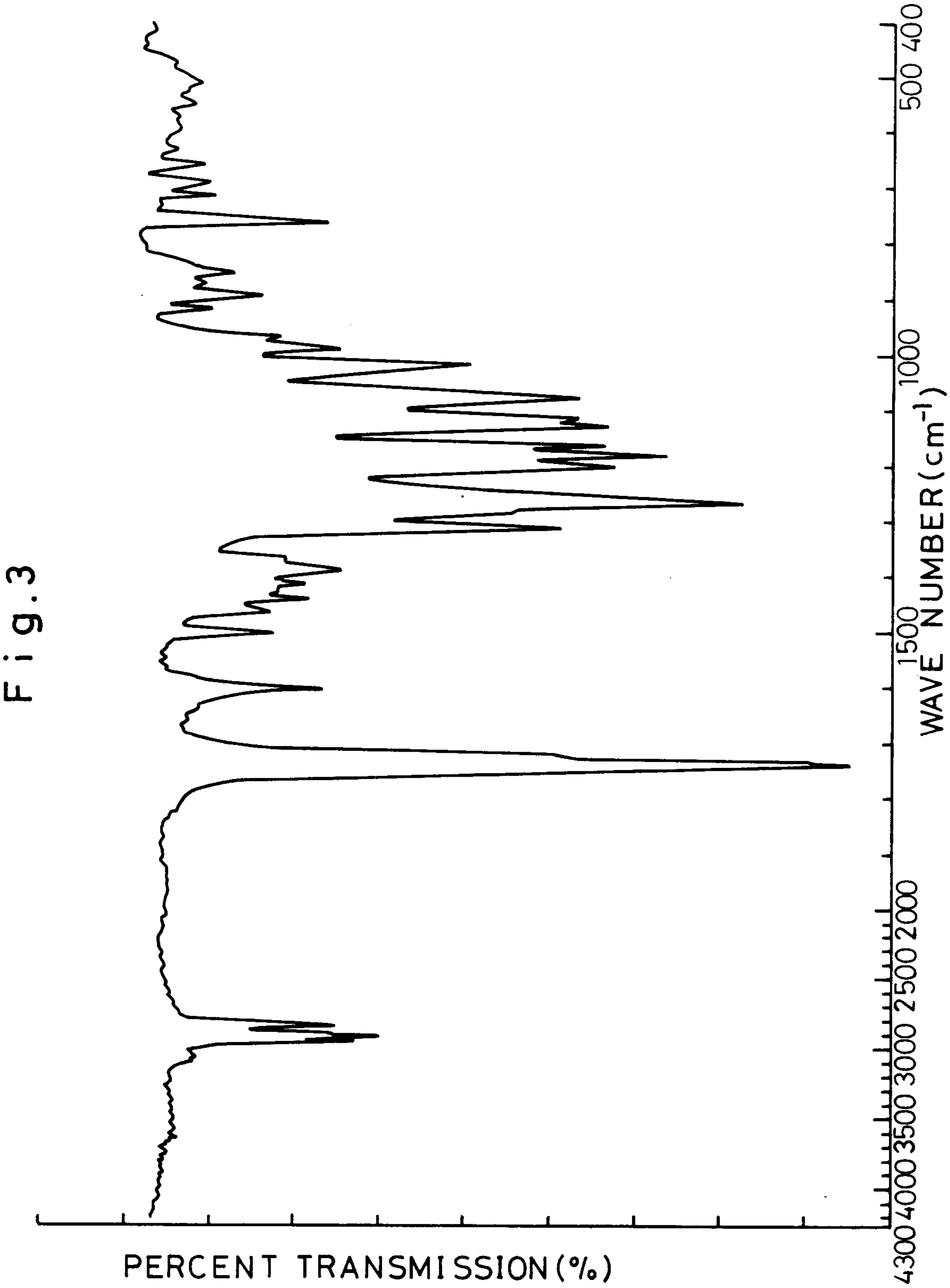
PERCENT TRANSMISSION (%)
WAVE NUMBER (cm⁻¹)
4300 4000 3500 3000 2500 2000 1500 1000 500 400

Fig.4

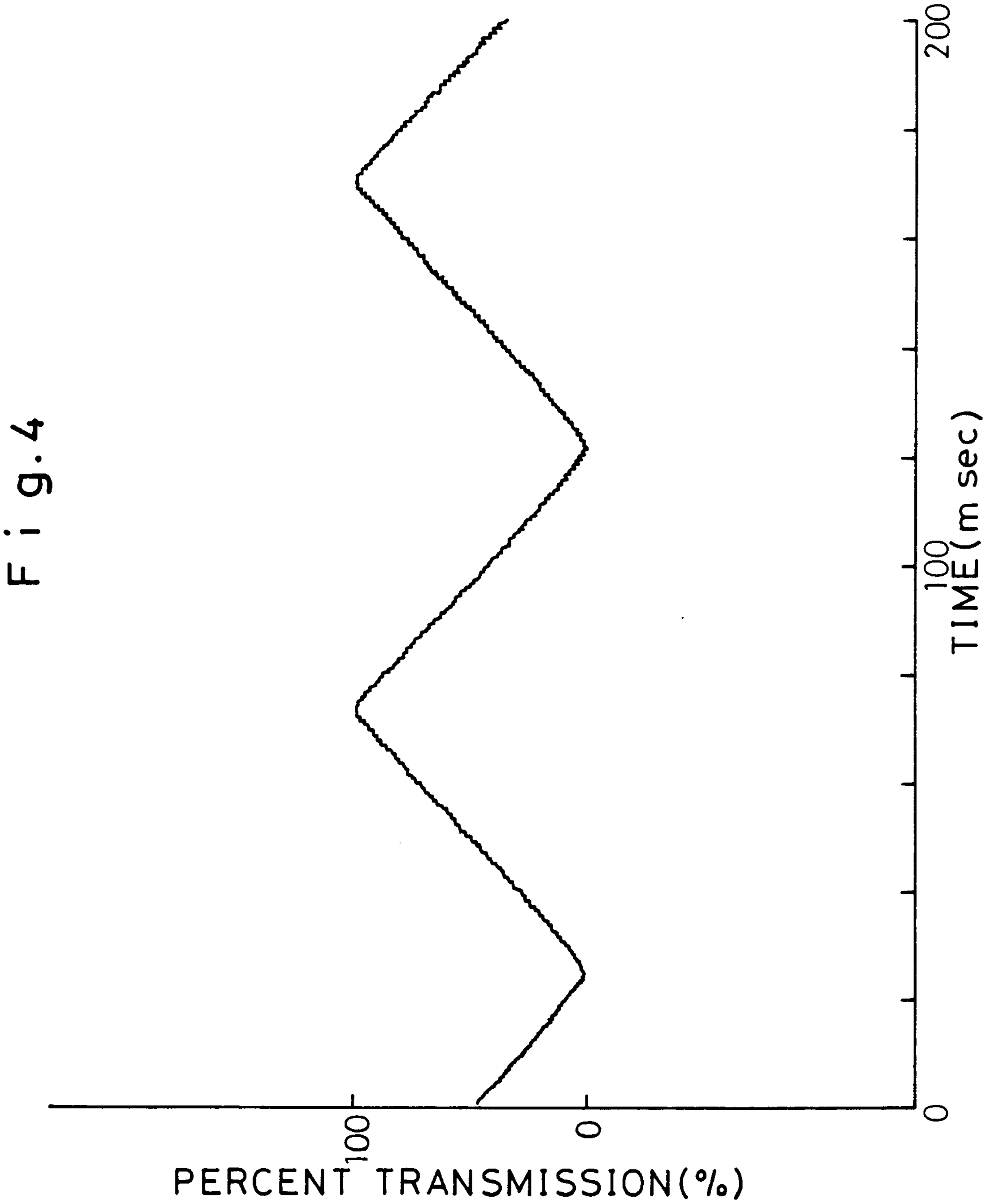

Fig.5